# EUROPÄISCHE PATENTSCHRIFT

(11) **EP 2 600 854 B1**
(45) Veröffentlichungstag und Bekanntmachung des Hinweises auf die Patenterteilung: **27.01.2016**
(21) Anmeldenummer: 11743433.2
(22) Anmeldetag: 06.07.2011
(51) Int. Cl.: A61K 8/35, A61P 17/00, A61Q 5/06, A61Q 5/12, A61Q 19/00, C07C 43/23, C07C 49/67, C07C 49/755, C07C 49/796, C07C 49/84

(54) **PHENETHYL-, PHENETHYLEN-, PHENETIN- UND INDANONDERIVATE**
PHENETHYL, PHENETHYLENE, PHENETHYNE AND INDANONE DERIVATES IN COSMETIC FORMULATIONS, ESPECIALLY FOR USE IN SKIN AGEING PROCESSES
DÉRIVÉS DE PHÉNYLÉTHYLE, DE PHÉNYLÉTHYLÈNE, DE PHÉNYLÉTHYNE ET D'INDANONE DANS DES PRÉPARATIONS COSMÉTIQUES, NOTAMMENT UTILISÉES POUR LUTTER CONTRE DES PROCESSUS DE VIEILLISSEMENT DE LA PEAU

(30) Priorität: 03.08.2010 DE 102010033138
(43) Veröffentlichungstag der Anmeldung: 12.06.2013
(73) Patentinhaber: Merck Patent GmbH, 64293 Darmstadt (DE)
(72) Erfinder: RUDOLPH, Thomas, 64291 Darmstadt (DE); BUEHLE, Philipp, 64291 Darmstadt (DE); SCHEURICH, René, Peter, 64846 Gross-Zimmern (DE)
(86) Internationale Anmeldenummer: PCT/EP2011/003359
(87) Internationale Veröffentlichungsnummer: WO 2012/016619

(56) Entgegenhaltungen:
- WO-A1-03/037315
- DD-A1- 69 672
- DE-A1- 19 847 778
- MCDONALD E ET AL: "Electrocyclic Reactions of Protonated Chalcones: Synthesis of 3-Aryl-indan-1-ones", JOURNAL OF THE CHEMICAL SOCIETY, PERKIN TRANSACTIONS 1, CHEMICAL SOCIETY, LETCHWORTH; GB, 1. Januar 1980 (1980-01-01), Seiten 837-842, XP009151978, ISSN: 0300-922X

## Beschreibung

Die Erfindung betrifft die Verwendung einer oder mehrerer Verbindungen, insbesondere die Verwendung von Phenethyl-, Phenethylen- und Phenethinderviaten, als Wirkstoffvorstufe in einer topischen Formulierung, dadurch gekennzeichnet, dass sich die Verbindung nach Applikation in einen Wirkstoff umwandelt, der in der Formulierung schlechter löslich ist als die Wirkstoffvorstufe, sowie Zusammensetzungen enthaltend Phenethyl-, Phenethylen, Phenethin- und Indanonderviate.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen.

Bei der Herstellung von Kosmetika stellen sich viele Herausforderungen. So wird von den Wirkstoffen einer kosmetischen Zubereitung zum Beispiel eine hohe Stabilität sowie gleichzeitig eine optimale Wirksamkeit erwartet. Weiterhin erwartet der Anwender beim Auftragen der Zubereitung auf die Haut ein angenehmes Hautgefühl.

Ein optimales Hautgefühl kann in der Regel dann erzielt werden, wenn verarbeitete Stoffe in der Zubereitung gelöst vorliegen. Durch etwaige Kristallisation eines Stoffes in der Zubereitung würde andernfalls das von der Zubereitung durch Applikation erzeugte Hautgefühl negativ beeinflusst.

Gleichzeitig ist jedoch die Penetration von verschiedenen Wirkstoffen durch die Haut häufig dann begünstigt, wenn der Wirkstoff partikulär in der Zubereitung vorliegt und als Partikel über den Haarfollikel in die Haut eingeschleußt werden kann. Demgegenüber weisen dermal applizierte Wirkstoffe in gelöster Form eine vergleichsweise schlechte Hautpenetration über den Haarfollikel auf (F. Knorr et. al., European Journal of Pharmaceutics and Biopharmaceutics 71 (2009) 173-180).

Die Aufgabe der vorliegenden Erfindung war somit das Bereitstellen von Substanzen, die in einer Formulierung beim Auftragen dieser ein ideales Hautgefühl erzeugen, zum anderen aber maximale Wirksamkeit auf Basis der Haarfollikelpenetration erzielen.

Das Problem wird dadurch gelöst, dass spezielle Wirkstoffvorstufen eingesetzt werden, die in der Zubereitung zunächst gelöst vorliegen. Erst nach Applikation wandeln sich die gelösten Vorstufen in partikuläre Wirkstoffe um. Diese Umwandlung kann z. B. tages- bzw. sonnenlichtinduziert erfolgen, bzw. durch den sauren pH-Wert des Hautschweißes katalysiert werden.

Ein erster Gegenstand der vorliegenden Erfindung ist daher die nichttherapeutische Verwendung einer oder mehrerer Verbindungen als Wirkstoffvorstufe in einer topischen Formulierung, dadurch gekennzeichnet, dass sich die Verbindung nach Applikation in einen Wirkstoff umwandelt, der in der Formulierung schlechter löslich ist als die Wirkstoffvorstufe, dadurch gekennzeichnet, dass als Wirkstoffvorstufe eine Verbindung, ausgewählt aus den Verbindungen der Formel 1, 2 und 3 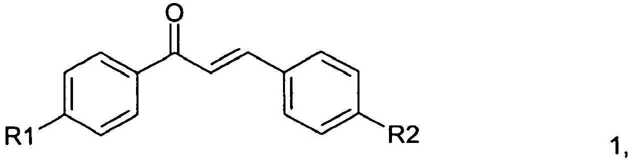 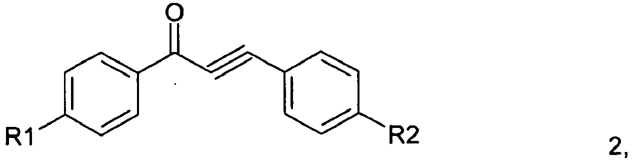 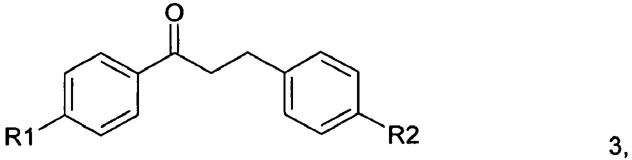 wobei R1 und R2 unabhängig voneinander für Methoxy, Isopropyl oder tert-Butyl stehen, verwendet wird.

Die Applikation bezeichnet das Auftragen oder Aufbringen der Formulierung auf die Haut oder Kopfhaut, welche von der gewählten Darreichungsform der Formulierung abhängt. So kann es sich dabei zum Beispiel bei einer Creme, einer Lotion oder einem Gel um das Verreiben oder Verteilen der Formulierung handeln, während eine Lösung oder ein Spray beispielsweise aufgesprüht werden kann.

Erfindungsgemäß bezeichnet der Begriff Wirkstoff eine Verbindung, die eine bestimmte Wirkung in einem Organismus hervorrufen kann. Insbesondere handelt es sich erfindungsgemäß um Substanzen, die den zeit- und/oder lichtinduzierten Alterungsprozessen der menschlichen Haut oder menschlichen Haaren entgegenwirken oder Substanzen, die zur Pflege, Konservierung oder Verbesserung des allgemeinen Zustands der Haut oder Haare beitragen. Im Speziellen kann es sich dabei beispielsweise um die Prophylaxe gegen oder die Reduktion von trockener Haut, Faltenbildung und/oder Pigmentstörungen, und/oder schädigender Effekte von UV-Strahlen auf die Haut handeln, sowie um die Prophylaxe gegen oder die Reduktion von Hautunebenheiten, wie Falten, feinen Linien, rauher Haut oder großporiger Haut. Weiterhin kann es sich erfindungsgemäß bevorzugt um Substanzen handeln, die hautaufhellende bzw. hautbräunende Wirkung aufweisen. Auch die Fähigkeit zur Stabilisierung anderer Formulierungsbestandteile wie UV-Filter, Farbstoffe und Vitamine kann eine bevorzugte Eigenschaft eines Wirkstoffs sein.

Dementsprechend wird unter dem Begriff Wirkstoffvorstufe erfindungsgemäß eine Verbindung verstanden, die als Vorstufe oder Precursor für einen Wirkstoff wie oben definiert fungiert.

Die dargestellte Strukturformel 1 umfasst erfindungsgemäß jeweils sowohl die trans- als auch die entsprechenden cis-Isomere, welche zum Beispiel photochemisch aus den trans-Isomeren gebildet werden können.

Bevorzugt werden Verbindungen ausgewählt aus den Verbindungen der Formel 1 oder 3 verwendet, besonders bevorzugt Verbindungen der Formel 1.

Bei dem entstehenden Wirkstoff handelt es sich bevorzugt um eine Verbindung der Formel 4 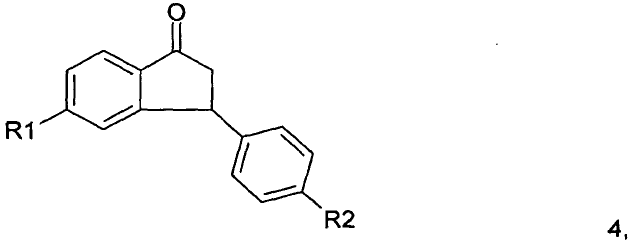 wobei R1 und R2 wie zuvor für Formel 1, 2 und 3 definiert sind.

Die Phenethylen-, Phenethin- und Phenethylderivate der Formeln 1, 2 und 3 können also als Wirkstoffvorstufen eingesetzt werden, die nach Applikation photoinduziert in die Wirkstoffverbindungen der Formel 4 überführbar sind.

Bei Betrachtung von Phenethin- (Formel 2) und Phenethylderivaten (Formel 3) ist zu berücksichtigen, dass diese nach topischer Applikation matrix-, licht- oder sauerstoffinduziert durch Oxidation der Phenethylderivate bzw. durch Reduktion der Phenethinderivate in die entsprechenden Phenethylenderivate (Formel 1) überführt werden können, die sich dann photoinduziert in Verbindungen der Formel 4 umwandeln können.

Die vorliegende Erfindung ermöglicht daher, Wirkstoffe gezielt in situ auf der Haut entstehen zu lassen. Diese Enstehung wird von situationstypischen Parametern wie UV-Strahlung oder Schweißentstehung getrieben. Gerade in diesen Situationen ist es häufig wichtig eine Antiphotoageingwirkung zu generieren oder das bestehende Schutzsystem aufrecht zu erhalten.

DE 19847778 A1 offenbart Verbindungen der Formel 1 als Zwischenstufen bei der Synthese von Dibenzoylmethanverbindungen.

Eine Umwandlung von Chalconen, insbesondere Phenethylenderivaten, in Arylindanone ist aus McDonald et al. bekannt (E. McDonald, P. Smith, J.C.S. Perkin I, 1980, 837-842). Hierin wird offenbart, dass die Cyclisierung eines Chalcons nach dessen Protonierung thermisch oder photochemisch induziert erfolgen kann.

WO 03/037315 offenbart Chalconverbindungen, die jedoch nicht gleichzeitig und ausschließlich an Position 4 und 4' substituiert sind.

Verbindungen der Formel 1 sind erfindungsgemäß besonders bevorzugt ausgewählt aus den Verbindungen der Formel 1 a-1 p:

| | | | |
|---|---|---|---|
| 1a | 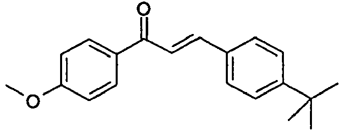 | 1b | 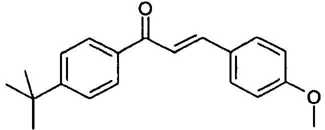 |
| 1c | 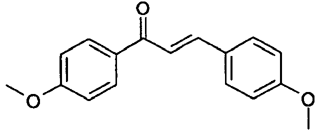 | 1d | 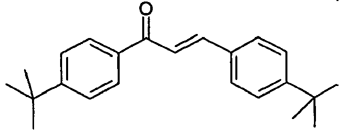 |
| 1k | 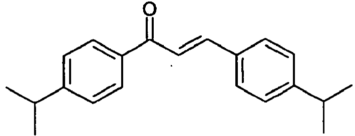 | | |
| 1m | 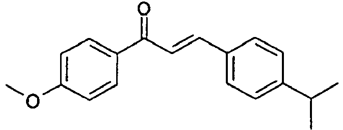 | 1n | 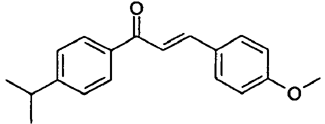 |
| 1o | 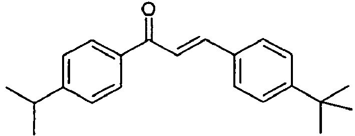 | 1p | 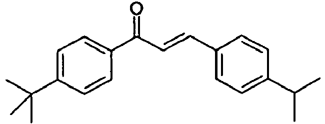 |

Besonders bevorzugt wird die Verbindung 1a eingesetzt.

Verbindungen der Formel 2 sind erfindungsgemäß besonders bevorzugt ausgewählt aus den Verbindungen der Formel 2a-2p:

| | | | |
|---|---|---|---|
| 2a | 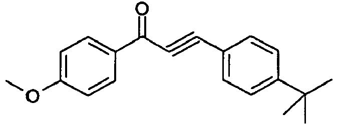 | 2b | 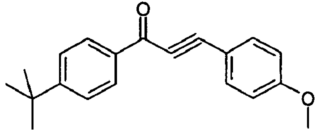 |
| 2c | 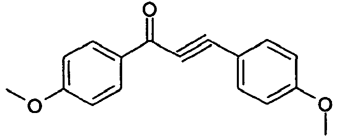 | 2d | 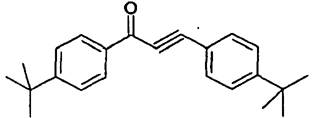 |
| 2k | 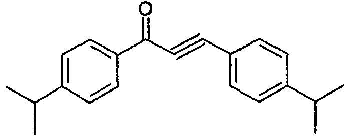 | | |
| 2m | 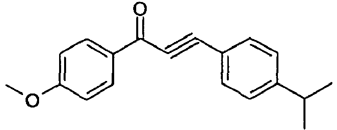 | 2n | 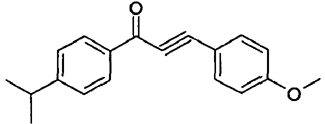 |
| 2o | 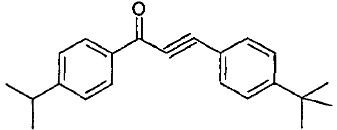 | 2p | 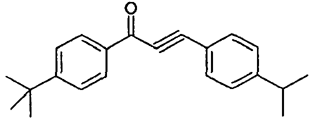 |

Verbindungen der Formel 3 sind erfindungsgemäß besonders bevorzugt ausgewählt aus den Verbindungen der Formel 3a-3p:

| | | | |
|---|---|---|---|
| 3a | 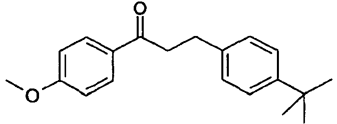 | 3b | 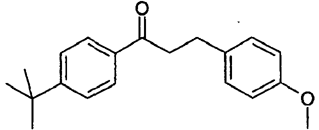 |
| 3c | 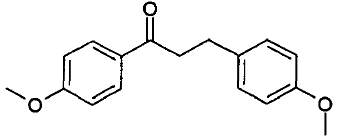 | 3d | 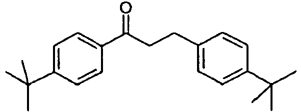 |
| 3k | 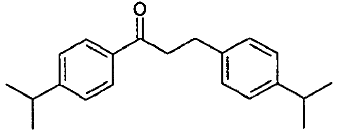 | | |

| | | | |
|---|---|---|---|
| 3m | 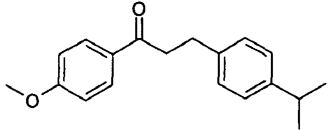 | 3n | 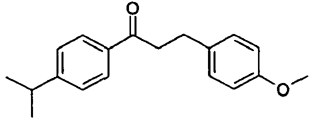 |
| 3o | 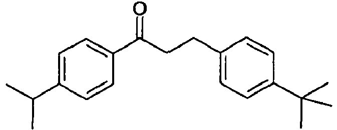 | 3p | 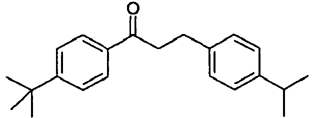 |

Verbindungen der Formel 4 sind erfindungsgemäß besonders bevorzugt ausgewählt aus den Verbindungen der Formel 4a-4p:

| | | | |
|---|---|---|---|
| 4a | 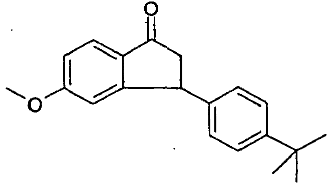 | 4b | 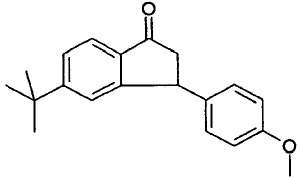 |
| 4c | 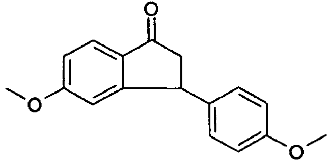 | 4d | 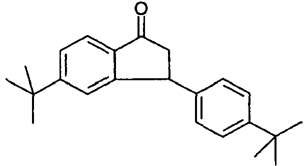 |
| 4k | 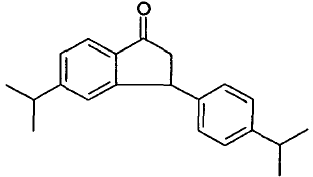 | | |
| 4m | 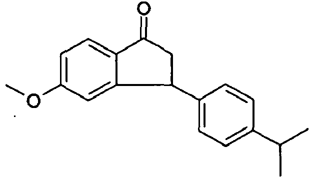 | 4n | 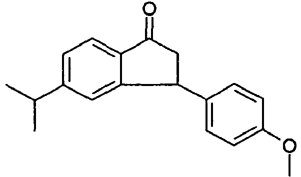 |
| 4o | 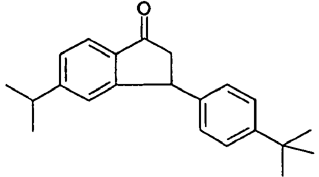 | 4p | 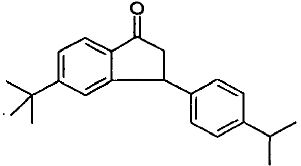 |

Die Substanzen der Formel 4 sind zum Beispiel geeignet, die Haut zu schützen und dem Hautalterungsprozeß entgegenzuwirken. Mechanistisch geht dies insbesondere auf die antioxidative Wirkung der Substanzen zurück. Weiterhin können sie hautaufhellende bzw. hautbräunende Wirkung aufweisen. Auch die Stabilisierung anderer Formulierungsbestandteile wie UV-Filter und Farbstoffe durch diese Substanzen ist denkbar.

Daneben können die Verbindungen der Formeln 1, 2 und 3 selbst Antiageing-Wirkung entfalten, die insbesondere in den äußeren Hautschichten stattfindet. Demgegenüber wirken über den Haarfollikel aufgenommene Wirkstoffe, insbesondere die Indanonderivate der Formel 4 in tieferen Hautschichten. Dies ist insbesondere deswegen wünschenswert, da nach neuesten Erkenntnissen auch langwellige Sonnenlichtstrahlung, insbesondere sichtbares Licht und Infrarotstrahlung, zu Hautschäden führen. Für langwellige Strahlung ist bekannt, dass diese über große Eindringtiefen in die Haut charakterisiert ist.

Verbindungen der Formel 1, 2 und 3 können insbesondere als Redoxpaare oder Redoxtriple eingesetzt werden. Die Einzelkomponenten innerhalb dieser Redoxpaare bzw. Redoxtriple unterscheiden sich lediglich im Vorhandensein von einer aliphatischen Doppel- oder Dreifachbindung. Beispielsweise bilden 1 a, 2a und 3a ein Redoxtriple. 1 a und 2a bilden demgegenüber ein Redoxpaar.

Damit sind durch die Verwendung erfindungsgemäßer Verbindungen bzw. erfindungsgemäßer Zusammensetzungen insbesondere folgende Vorteile gegeben:
- Verbindungen der Formel 4 weisen eine antioxidante Wirkung gegen Radikale auf, die z.B. durch UV-Licht oder durch thermolytische Prozesse, wie dem Rauchen, induziert werden, wie z.B. gegen das Superoxidradikalanion oder das NO-Radikal, bzw. gegen reaktive Sauerstoffspezies, wie z. B. gegen Singulettsauerstoff und Peroxide;
- Verbindungen der Formel 4 vereinigen in sich eine starke antioxidative Aktivität in Kombination mit einer hohen molekularen Stabilität gegen Umwelteinflüsse wie Wärme oder Licht, insbesondere UV-Licht;
- Eine produktstabilisierende Wirkung auf kosmetische, pharmazeutische, insbesondere dermatologische Produkte, insbesondere solche, die Farb-, Konsistenz-, Geruchsstoffe oder Vitamine enthalten;
- Die Verbindungen der Formel 1, 2 und 3 weisen ähnlich hervorragende antioxidative Eigenschaften wie Verbindungen der Formel 4 auf, wobei sich diese Wirkweise aufgrund der größeren Lipophilie im Vergleich zu Verbindungen der Formel 4 vornehmlich in oberen Haut- bzw. Haarschichten, bzw. an Haut- und Haaroberflächen entfaltet;
- Verbindungen der Formeln 1, 2, 3 und 4 können auch den Haut- bzw. Haarton positiv beeinflussen, wobei die Wirkweisen unterschiedlich sind. So unterstützen Verbindungen der Formel 1, 2 oder 3 überwiegend den nicht-enzymatischen Bräunungsprozess durch Selbstbräuner in Oberflächennähe (z. B. Schutz von Dihydroxyaceton vor Autoxidation), während Verbindungen der Formel 4 überweigend auf den enzymatischen Bräunungsprozess der Melanogenese Einfluss nehmen;
- Bevorzugte Verbindungen der Formeln 1, 2, 3 und 4 eignen sich zum DNA-Schutz. Sie können zudem die Zellproliferation und Zelldifferenzierung in der Haut fördern und so z. B. zur Anti-Ageing, Anti-Falten und wundheilenden Wirkung beitragen;
- Bevorzugte Verbindungen der Formeln 1, 2, 3 und 4 eignen sich zur Erzeugung bzw. Erhöhung ("boost"-Effekt) von Lichtschutzfaktoren, wie LSF, SPF, PPD oder IPD, oder Radikalschutzfaktoren;
- Eine stabilisierende Wirkung auf autooxidierbare Polyethylenglycol (PEG)- oder Polyglycerin (PG)-Derivate, wie insbesondere PEG- oder PGhaltige Emulgatoren, wie sie weiter unten in dieser Anmeldung genannt werden, bzw. eine Reduktion der schädigenden Wirkung der Abbauprodukte autooxidierbarer Polyethylenglycol (PEG)- oder Polyglycerin (PG)-Derivate;
- Eine stabilisierende Wirkung auf Farb-, Konsistenz- oder Geruchsstoffe, oder auf Antioxidantien oder Vitamine, und UV Filter sowie Titandioxidhaltige Pigmente, insbesondere in kosmetische, pharmazeutische, insbesondere dermatologische Produkten;
- Je nach antioxidativem Potential und Wirkort eignen sich Verbindungen der Formeln 1 bis 4 dazu, den Hautfarbton aufzuhellen. Insbesondere Verbindungen der Formeln 2 und 3 zeichnen sich dadurch aus, dass sie die lichtbedingte Bräunungsinduktion hemmen;
- Verbindungen der Formeln 1 bis 4, insbesondere die an der Oberfläche wirkenden Verbindungen der Formel 1 bis 3, können als Anti-Acne oder Anti-Schuppenmittel Verwendung finden. Im Allgemeinen lassen sich auch antimikrobielle und konservierungsfördernde Wirkungen erzielen;
- Aufgrund ihrer Tiefenwirkung eignen sich insbesonder Verbindungen der Formel 4 als Anti-Cellulitewirkstoffe;

Zusätzlich sind bevorzugte der hier beschriebenen Verbindungen farblos oder nur schwach gefärbt und führen so nicht oder nur in geringer Weise zu Verfärbungen der Zubereitungen.

Die folgenden Schemata 1 und 2 veranschaulichen mögliche Herstellungswege für die Verbindungen der Formeln 1, 2 und 3. Verbindungen der Formel 4 können wie bereits beschrieben photoinduziert aus Verbindungen der Formel 1 gewonnen werden.

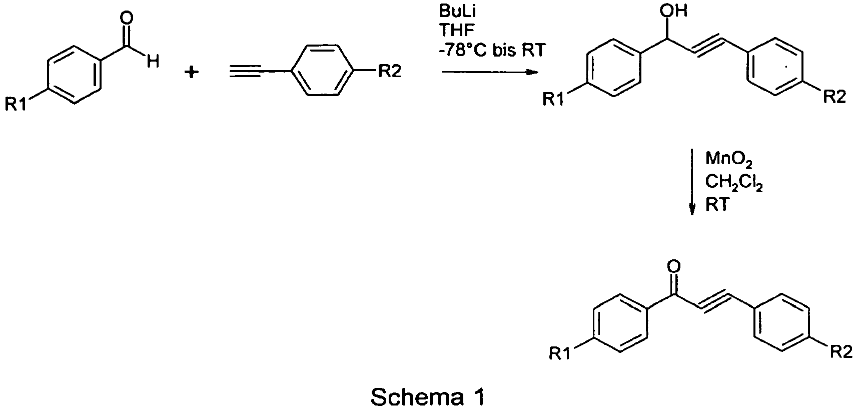

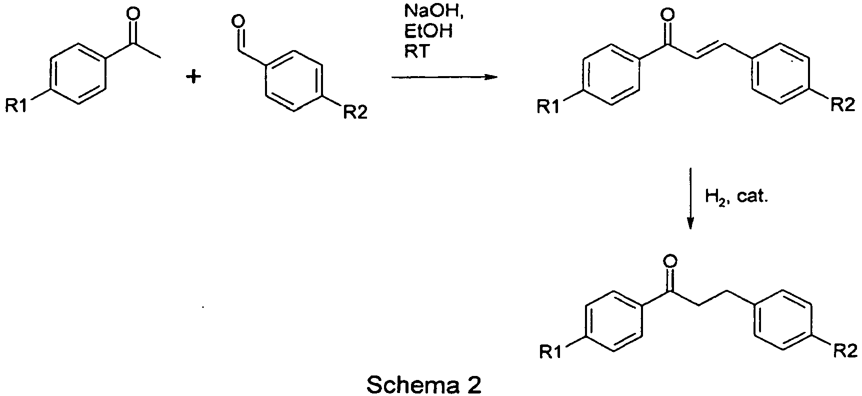

Gemäß der vorliegenden Erfindung steht Alkyl als Abkürzung für Alkylgruppe. Der Alkylrest kann geradkettig oder verzweigt sein.

Der C3-C4-Alkylrest ist beispielsweise Isopropyl, Propyl, Butyl, sek.-Butyl oder tert.-Butyl.

Bei dem C1-C8-Alkylrest kann es sich neben den oben gelisteten C3-C4-Alkylresten beispielsweise auch um Methyl, Ethyl, Pentyl, 1-, 2- oder 3-Methylbutyl, 1,1-, 1,2- oder 2,2-Dimethylpropyl, 1-Ethylpropyl, Hexyl, Heptyl, 1-Ethyl-pentyl, Octyl oder 1-Ethyl-hexyl handeln.

Der C1-C20-Alkylrest ist zusätzlich beispielsweise Nonyl, Decyl, Undecyl, Dodecyl, Tridecyl, Tetradecyl, Pentadecyl, Hexadecyl, Heptadecyl, Octadecyl, Nonadecyl oder Eicosyl.

Alkoxy steht als Abkürzung für Alkoxygruppe. Der Alkoxyrest kann linear oder verzweigt sein.

Beispielsweise handelt es sich bei der C1-C4-Alkoxygruppe um Methoxy, Ethoxy, Propoxy, Isopropoxy, Butoxy, Isobutoxy oder Tert.-Butoxy. Beispiele für C1- bis C20-Alkoxygruppen sind ferner 1,1-, 1,2- oder 2,2-Dimethylpropoxy, 1-Ethylpropoxy, Hexoxy, Heptoxy, 1-Ethyl-pentoxy, Octoxy, Nonoxy, Decoxy, Undecoxy, Dodecoxy, Tridecoxy, Tetradecoxy, Pentadecoxy, Hexadecoxy, Heptadecoxy, Octadecoxy oder Nonadecoxy.

Gegenstand der vorliegenden Erfindung ist auch eine kosmetische Zubereitung, enthaltend mindestens eine Verbindung der Formel 1, 2 und/oder 3 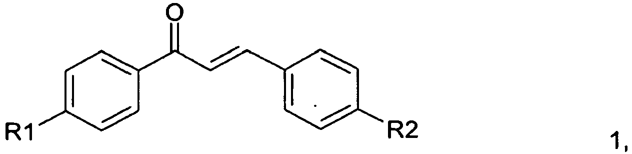 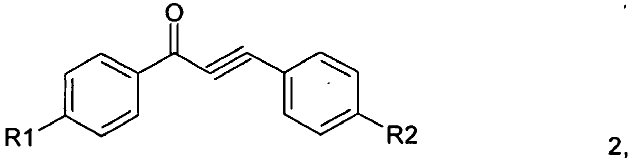 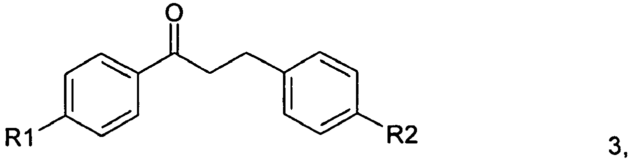 wobei R1 und R2 unabhängig voneinander für Methoxy, Isopropyl oder tert-Butyl stehen und wobei sich die mindestens eine Verbindung der Formel 1, 2, und/oder 3 nach Applikation in einen Wirkstoff umwandelt, der in der Formulierung schlechter löslich ist als die mindestens eine Verbindung der Formel 1, 2 und/oder 3.

Dabei sind die Verbindungen der Formel 1, 2 und 3 jeweils besonders bevorzugt ausgewählt aus den Verbindungen der Formel 1a bis 1 p, 2a bis 2p und 3a bis 3p wie oben definiert.

Erfindungsgemäß vorteilhaft sind Zubereitungen enthaltend Verbindungen der Formel 1 und/oder 3, insbesondere Verbindungen der Formel 1.

In einer besonders bevorzugten Ausführungsform der vorliegenden Erfindung werden Verbindungen der Formel 1 a eingesetzt.

Die Substanzen der vorliegenden Erfindung können dabei in den Formulierungen auch miteinander kombiniert werden, und teilweise sogar ineinander umgewandelt werden.

Bei den Zubereitungen handelt es sich dabei üblicherweise um topisch anwendbare Zubereitungen, beispielsweise kosmetische Formulierungen. Die Zubereitungen enthalten in diesem Fall einen kosmetisch oder dermatologisch geeigneten Träger und je nach gewünschtem Eigenschaftsprofil optional weitere geeignete Inhaltsstoffe.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Gegenstand der vorliegenden Erfindung ist auch ein Verfahren zur Herstellung einer Zubereitung, wie zuvor beschrieben, dadurch gekennzeichnet, dass mindestens eine Verbindung der Formel 1, 2 und/oder 3 mit einem Träger und gegebenenfalls mit weiteren Aktiv- oder Hilfsstoffen vermischt wird. Geeignete Trägerstoffe sowie Aktiv- oder Hilfsstoffe sind im nachfolgenden Teil ausführlich beschrieben.

In bevorzugten Ausführungsformen wird die mindestens eine Verbindung der Formel 1, 2 und/oder 3 mit den definierten oder als bevorzugt angegebenen Substituenten oder bevorzugte Einzelverbindungen in den erfindungsgemäßen Zubereitungen typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt. Dabei bereitet es dem Fachmann keinerlei Schwierigkeiten die Mengen abhängig von der beabsichtigten Wirkung der Zubereitung entsprechend auszuwählen.

Der Zusatz von Verbindungen, wie sie in WO 2007/121818 A1 offenbart werden, zu den erfindungsgemäßen Formulierungen ist besonders vorteilhaft, da dies eine Wirkverstärkung ermöglicht. Daher sind auch Zubereitungen Gegenstand der vorliegenden Erfindung, die zusätzlich eine oder mehrere Verbindungen der Formel 5 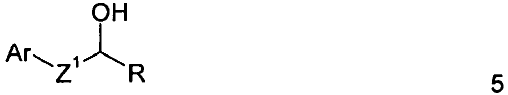 wobei R für 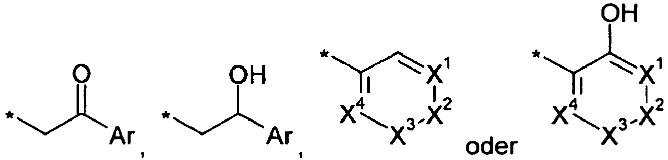 steht, wobei
Ar jeweils unabhängig voneinander stehen für einen unsubstituierten oder ein- oder mehrfach substituierten aromatischen Ring oder kondensierte Ringsysteme mit 6 bis 18 C-Atomen, von denen mindestens ein Ring aromatischen Charakter besitzt, worin pro Ring auch ein oder zwei CH-Gruppen durch C=O, N, O oder S ersetzt sein können und in einem kondensierten Ringsystem auch ein oder zwei CH₂-Gruppen durch C=O oder C=CH₂ ersetzt sein können,
Z¹ steht für CR⁷R⁸ oder eine Einfachbindung,
X¹ bis X⁴ jeweils unabhängig voneinander ausgewählt sind aus C-R¹, O, N oder S, wobei 2 benachbarte Reste aus X¹ bis X⁴ gemeinsam auch stehen können für einen unsubstituierten oder ein- oder mehrfach substituierten Ring oder kondensierte Ringsysteme mit 6 bis 18 C-Atomen, von denen vorzugsweise mindestens ein Ring aromatischen Charakter besitzt, worin pro Ring auch ein oder zwei CH-Gruppen durch C=O, N, O oder S ersetzt sein können und in einem kondensierten Ringsystem auch ein oder zwei CH₂-Gruppen durch C=O oder C=CH₂ ersetzt sein können,
R¹ ausgewählt ist aus H,
geradkettigen oder verzweigten C₁- bis C₂₀-Alkoxygruppen, wobei die Alkylketten jeweils auch durch Sauerstoff oder Stickstoff unterbrochen sein können,
geradkettigen oder verzweigten C₁- bis ₂₀-Alkylgruppen, wobei die Alkylketten jeweils auch durch Sauerstoff oder Stickstoff unterbrochen sein können,
geradkettigen oder verzweigten C₃- bis C₂₀-Alkenylgruppen, geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylketten jeweils auch durch Sauerstoff oder Stickstoff unterbrochen sein können,
geradkettigen oder verzweigten C₁- bis C₂₀-Hydroxyalkoxygruppen, wobei die Hydroxygruppe(n) an primäre oder sekundäre Kohlenstoffatome der Kette gebunden sein können und weiter die Alkylkette auch durch Sauerstoff unterbrochen sein kann, geradkettigen oder verzweigten C₁- bis C₂₀-Alkylaminogruppen, geradkettigen oder verzweigten C₁- bis C₂₀-Dialkylaminogruppen, oder R¹ steht für eine Carbonsäure-, Phosphorsäure-, Sulfonsäure-, Schwefelsäure- oder Sulfonfunktion, die optional mit geradkettigen oder verzweigten C₁- bis ₂₀-Alkylgruppen bzw. geradkettigen oder verzweigten C₃- bis C₂₀-Alkenylgruppen verestert oder alkyliert sein kann,
R⁷ und R⁸ jeweils unabhängig voneinander ausgewählt sind aus H, OH, geradkettigen oder verzweigten C₁- bis C₂₀-Alkoxygruppen, geradkettigen oder verzweigten C₁- bis ₂₀-Alkylgruppen, geradkettigen oder verzweigten C₃- bis C₂₀-Alkenylgruppen, geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, geradkettigen
oder verzweigten C₁- bis C₂₀-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, oder deren Salze enthalten.

Bevorzugt sind die Verbindungen der Formel 5 ausgewählt aus den Verbindungen der Formel 5a und/oder 5b 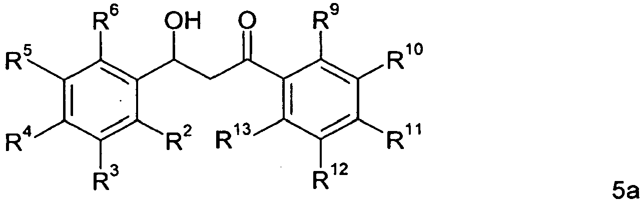 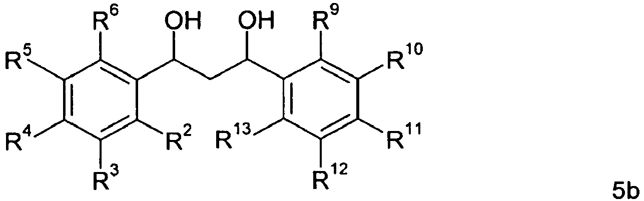 wobei
R⁴ und R¹¹ jeweils unabhängig voneinander H, geradkettige oder verzweigte C1- bis C20-Alkylgruppe, geradkettige oder verzweigte C1- bis C20-Alkoxygruppe oder geradkettige oder verzweigte C1- bis C20-Dialkylaminogruppe,
R⁶ H oder eine Carbonsäure-, Phosphorsäure-, Sulfonsäure-, Schwefelsäure- oder Sulfonfunktion, die mit geradkettigen oder verzweigten C1- bis C20-Alkylgruppen oder geradkettigen oder verzweigten C3- bis C20-Alkenylgruppen verestert oder alkyliert sein kann und R², R³, R⁵, R⁹, R¹⁰, R¹² und R¹³ H bedeuten.

Besonders bevorzugt sind die Verbindungen der Formel 5a und 5b ausgewählt aus den Verbindungen umfassend 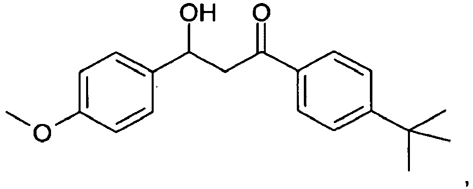 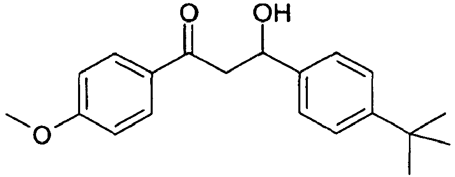 und

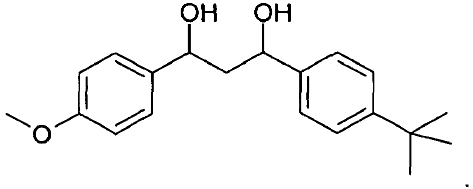

In den beschriebenen Zubereitungen, die erfindungsgemäß mindestens eine Verbindung der Formel 1, 2 und/oder 3 enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen enthalten neben den Verbindungen der Formel 1, 2 und/oder 3 sowie den gegebenenfalls anderen Inhaltsstoffen Selbstbräunungssubstanzen und/ oder UV-Filter.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.
Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.
β,β-Diphenylacrylate Derivate: Octocrylene, z. B. vetrieben unter dem Namen "Eusolex® OCR" von der Firma Merck", "Uvinul N539" von der Fa. BASF, Etocrylene, z. B. vetrieben unter dem Namen "Uvinul N35" von der BASF.
Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40"; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.
Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.
Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.
Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. BASF.
Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V.
Anthranilin Derivate: Menthyl anthranilate, z. B. vetrieben unter dem Namen "Neo Heliopan MA" von der Fa. Symrise.
Imidazol Derivate: Ethylhexyldimethoxybenzylidenedioxoimidazoline propionat.
Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.
4,4-Diarylbutadien Derivate: 1,1-Dicarboxy(2,2'-dimethylpropyl)-4,4-diphenylbutadiene.
Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung 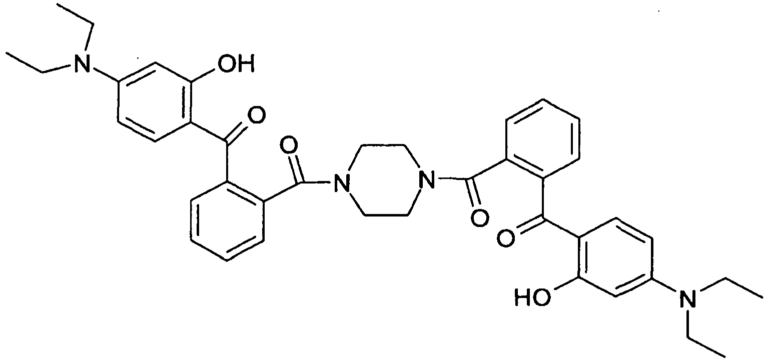 oder die UV-Filter der folgenden Strukturen 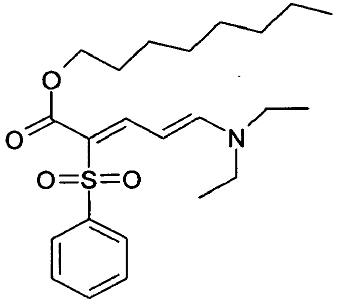 oder

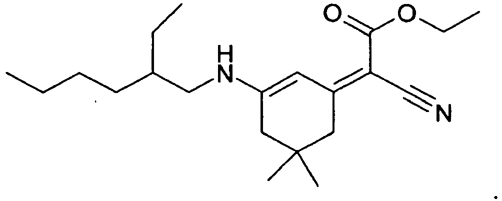

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

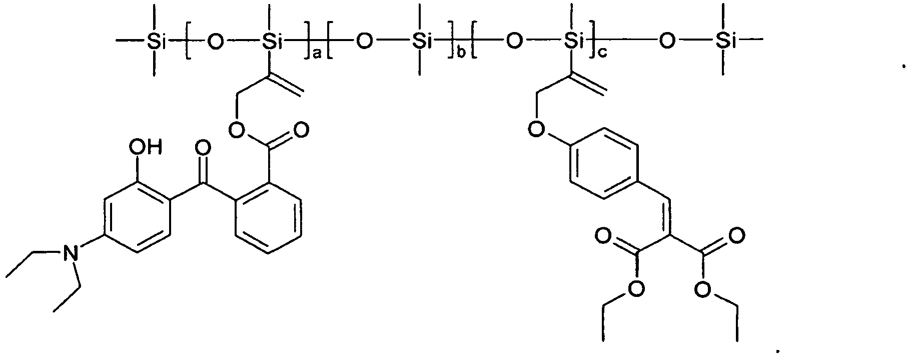

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Bevorzugte Zubereitungen enthalten neben den Verbindungen der Formel 1, 2 und/oder 3 sowie den gegebenenfalls organischen UV-Filtern, wie zuvor beschrieben, weitere anorganische UV-Filter, sogenannte partikuläre UV-Filter.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B. das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben. Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Kapselwände können auch aus PMMA bestehen. Besonders bevorzugt einzusetzende Kapseln weisen Wände auf, die durch einen SolGel-Prozess, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

Ferner können die erfindungsgemäßen Zubereitungen mindestens einen weiteren Selbstbräuner als weiteren Inhaltsstoff enthalten.

Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Bevorzugte Zubereitungen können ebenfalls mindestens einen weiteren kosmetischen Wirkstoff enthalten, beispielsweise ausgewählt aus Antioxidantien, Anti-aging-Wirkstoffen, Anti-Falten, Anti-Schuppen, Anti-Akne, Deodorants, Anti-Cellulite Wirkstoffen, Selbstbräunungssubstanzen, hautaufhellenden Wirkstoffen oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen-methionin), Stilbene und deren Derivate (z.B. Stilbenoxid, trans-Stilbenoxid).

Geeignete Antioxidantien sind auch Verbindungen der Formeln A oder B 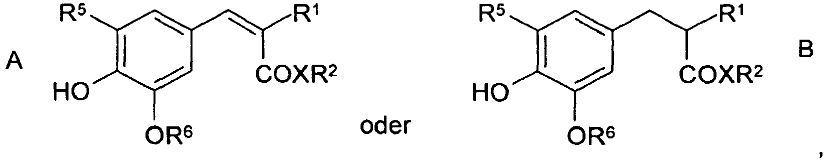 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,

vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), ß-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-aging Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die Zubereitungen können auch ein oder mehrere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin, Niacinamide, Azelainsäure, Elaginsäure, Maulbeerbaumextract, Magnesium-ascorbyl-phosphat, Süßholzwurzelextrakt, Emblica, Ascorbinsäure oder Rucinol.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-CelluliteWirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrig-alkoholischer Gele bzw. Lösungen auf die Haut aufgesprüht werden kann. Sie können als feste Stifte vorliegen oder als Aerosol konfektioniert sein.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butytstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder -monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycoi(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat,
Polyethylenglycol(21)glyceryllaurat, Polyethylenglycol(22)glyceryllaurat,
Polyethylenglycol(23)glyceryllaurat,
Poiyethylenglycol(6)glycerylcaprat/cprinat,
Polyethylenglycol(20)glyceryloleat, Polyethylenglycol(20)glycerylisostearat,
Polyethylenglycol(18)glyceryloleat(cocoat) zu wählen.

Es ist ebenfalls günstig, die Sorbitanester aus der Gruppe
Polyethylenglycol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane, bevorzugt Alkane.

Ein weiterer Gegenstand der vorliegenden Erfindung sind außerdem Verbindungen, ausgewählt aus den Verbindungen umfassend

| | | | |
|---|---|---|---|
| 2a | 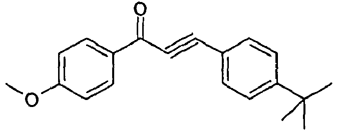 | 2b | 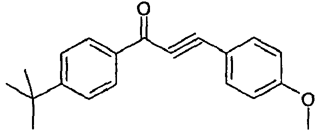 |
| 2k | 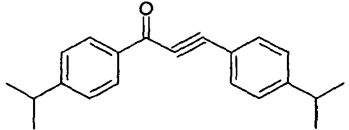 | 2m | 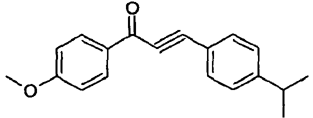 |
| 2n | 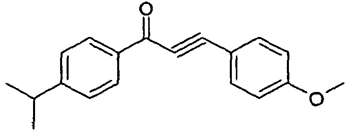 | 2o | 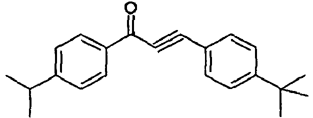 |
| 2p | 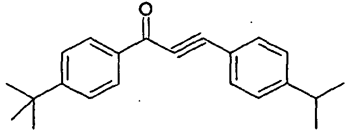 | | |
| 3d | 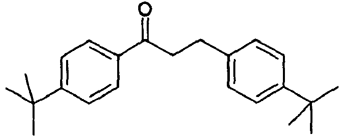 | 3k | 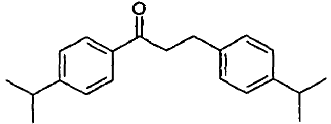 |
| 3o | 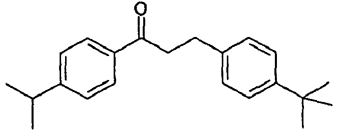 | 3p | 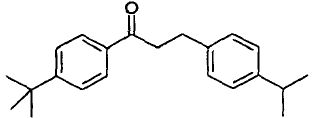 |

| | | | |
|---|---|---|---|
| 4a | 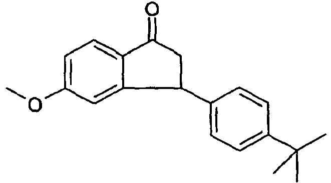 | 4b | 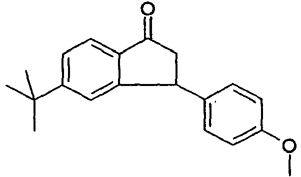 |
| 4d | 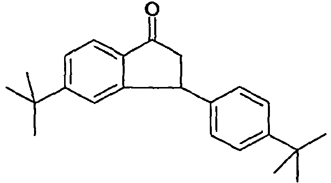 | | |
| | | 4k | 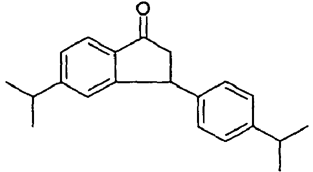 |
| 4m | 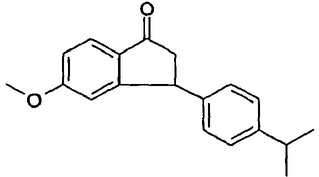 | 4n | 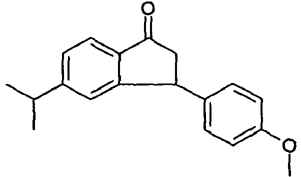 |
| 4o | 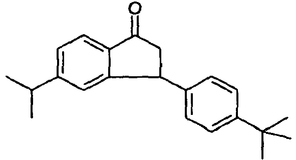 | 4p | 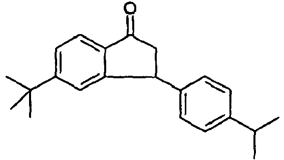 |

Wie bereits zuvor beschrieben weisen die erfindungsgemäßen Verbindungen viele Vorteile auf: Verbindungen der Formel 2 oder 3 können z. B. gegen die zeit- oder lichtinduzierte Alterung der Haut eingesetzt werden. Erfindungsgemäße Verbindungen der Formel 4 sind ebenso geeignet, dem Alterungsprozess der Haut entgegen zu wirken. Ferner können diese Substanzen als Antioxidantien, sowie als hautbräunende oder hautaufhellende Substanzen eingesetzt werden.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Die im Folgenden angeführten Beispiele für den erfindungsgemäßen Gegenstand dienen lediglich der Erläuterung und engen die vorliegende Erfindung keineswegs in irgendeiner Weise ein. Im Übrigen ist die beschriebene Erfindung im gesamten beanspruchten Bereich ausführbar. Alle Verbindungen oder Komponenten, die in den Zubereitungen verwendet werden können, sind entweder bekannt und käuflich erhältlich oder können nach bekannten Methoden synthetisiert werden. Es werden in der Regel die INCI-Namen der verwendeten Rohstoffe angegeben (die INCI-Namen werden definitionsgemäß in englischer Sprache angegeben).

### Beispiele:

### Beispiel 1:

### Herstellung von E-3-(4-tert.-Butyl-phenyl)-1-(4-methoxy-phenyl)-propenon (1a) :

5,76g (144 mmol) NaOH-Plätzchen werden in einem Gemisch aus 130mL Wasser und 565 mL Ethanol gelöst. Danach werden 21,6g (144 mmol) 4-Methoxyacetophenon bei RT zugegeben. Anschließend wird der 4-tert.-Butyl-Benzaldehyd hinzugefügt und über Nacht gerührt. Dann wird der pH-Wert mit 2N HCl auf einen Wert von 3 eingestellt. Die Suspension wird auf 1°C abgekühlt, 1,5h bei dieser Temperatur nachgerührt und dann filtriert. Der Rückstand wird mit Heptan (2x25mL) gewaschen und trockengesaugt. Nach Trocknung i. Vak. bei 40°C werden 63,9g (125 mmol, 87%) Chalcon 1 a als hellgelbe Kristalle erhalten.

### Herstellung von 3-(4-tert.-Butyl-phenyl)-5-methoxy-indan-1-on (4a):

30,0g Polyphosphorsäure werden bei 100°C in 180mL wasserfreiem Toluol gelöst. Dann wird eine Lösung von 30,0g (102mmol) Chalcon 1a in 280mL wasserfreiem Toluol so zugetropft, dass die Temperatur im Reaktionsgefäß nicht unter 95°C sinkt. Es wird 42h bei 100°C gerührt, dann auf RT abgekühlt. Der Überstand wird abdekantiert und auf 600g Eiswasser gegeben. Nach Phasentrennung wird die wässrige Phase mit Toluol (2x100 mL) extrahiert. Die organische Phase wird mit weiteren 100mL Toluol verdünnt und mit Wasser (2x150mL) gewaschen und über Natriumsulfat getrocknet. Nach Filtration wird das Natriumsulfat noch mit Toluol (2x100mL) nachgewaschen und dann das Lösemittel im Vakuum entfernt. Der Rückstand wird aus Toluol/Heptan (120mL, 1:1) bei -20°C umkristallisiert. Nach Absaugen wird das Kristallisat mit Heptan (4x25mL) gewaschen. Nach Trocknung werden 17,8g (60,0 mmol9, 59%) der Substanz 4a als farblose Kristalle erhalten.

### Beispiel 2:

### Löslichkeitsvergleich:

20mg von Substanz 1 a werden mit 800µL des kosmetischen Öles Isopropylmyristat bei 30°C vollständig gelöst. Im Parallelversuch werden 20mg der Substanz 4a mit zunächst 800µL Isopropylmyristat versetzt. Dabei kann keine vollständige Löslichkeit erzielt werden. Selbst nach Zugabe weiterer 1200µL verbleibt für 4a ein deutlicher Bodensatz in der trüben Dispersion. Daraus errechnen sich Löslichkeiten für 1 a von mindestens 2,5% bzw. für 4a von maximal 1,0% bezogen auf das eingesetzte Volumen Öl. Im anschließenden Verifizierungsexperiment bei Raumtemperatur von 23°C ergeben sich Löslichkeiten von 2,4% für 1 a sowie 0,4% für 4a.

### Beispiel 3:

### In-situ Erzeugung von 4a:

5mg der Substanz 1 a werden in 10 mL Ethanol gelöst. Mit der Lösung wird ein NMR-Röhrchen vollständig befüllt, verschlossen und für 4 Stunden im Suntester sonnensimulierter Strahlung ausgesetzt (Atlas CPS+ Suntester, 765 Wm⁻²). Nach Bestrahlung wird die Lösung per HPLC untersucht. Im Dunkelexperiment ist die Substanz 4a abwesend, während im Belichtungexperiment Substanz 4a zu 2% bei der Detektionswellenlänge von 272nm nachgewiesen wird.

### Beispiel 4:

### Zubereitungen:

Im folgenden werden beispielhaft Rezepturen für kosmetische Zubereitungen angegeben. Entsprechende Zubereitungen können in gleicher Weise mit allen erfindungsgemäßen Verbindungen hergestellt werden.

Insbesondere können die in nachfolgenden Rezepturbeispielen verwendeten Substanzen 1a, 2a, 3a und 4a durch die ensprechenden Substanzen 1b, 2b, 3b und 4b ausgetauscht werden.

Es sind die INCI-Bezeichnungen der handelsüblichen Verbindungen angegeben.

UV-Pearl, OMC steht für die Zubereitung mit der INCI-Bezeichnung: Water (for EU: Aqua), Ethylhexyl Methoxycinnamate, Silica, PVP, Chlorphenesin, BHT. Diese Zubereitung ist im Handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht ist.

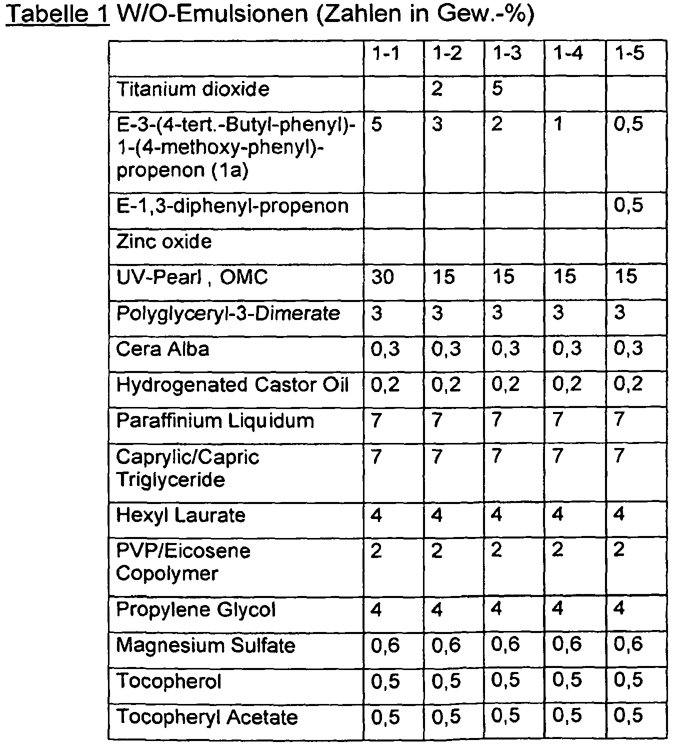

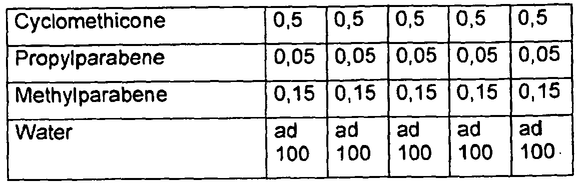

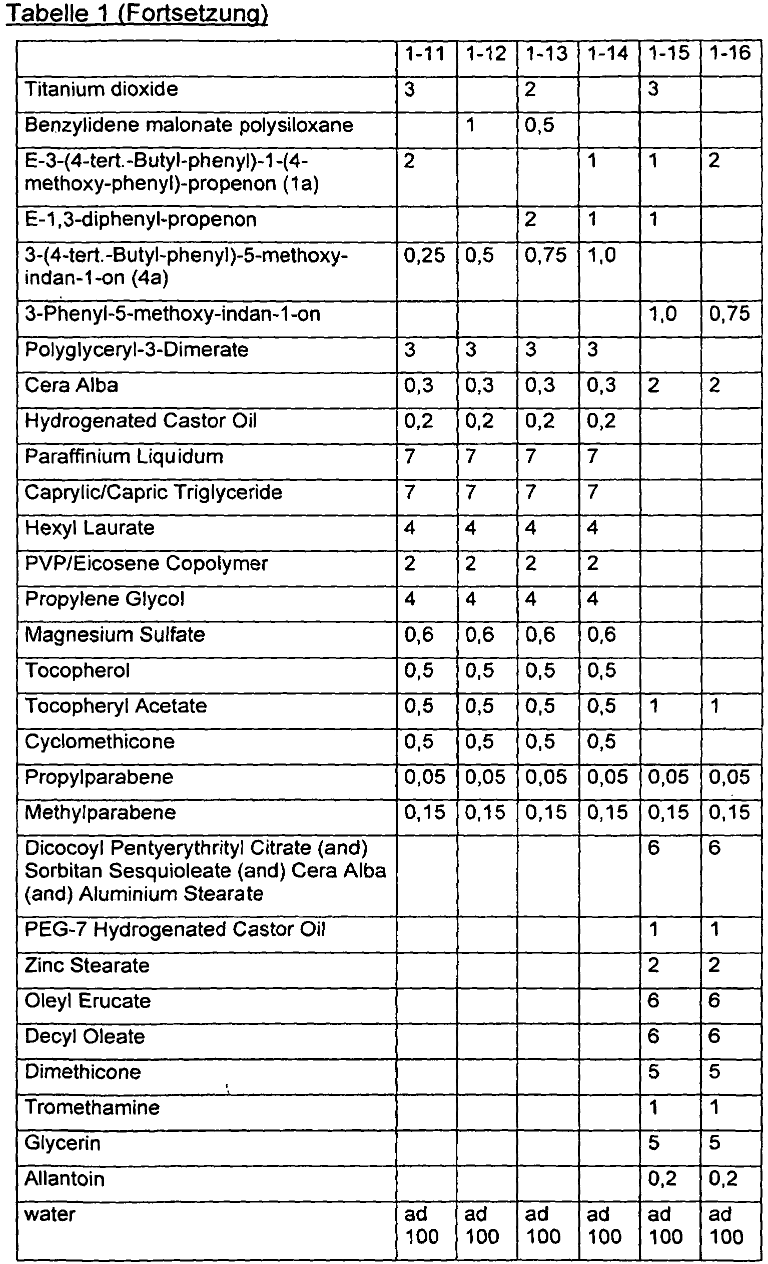

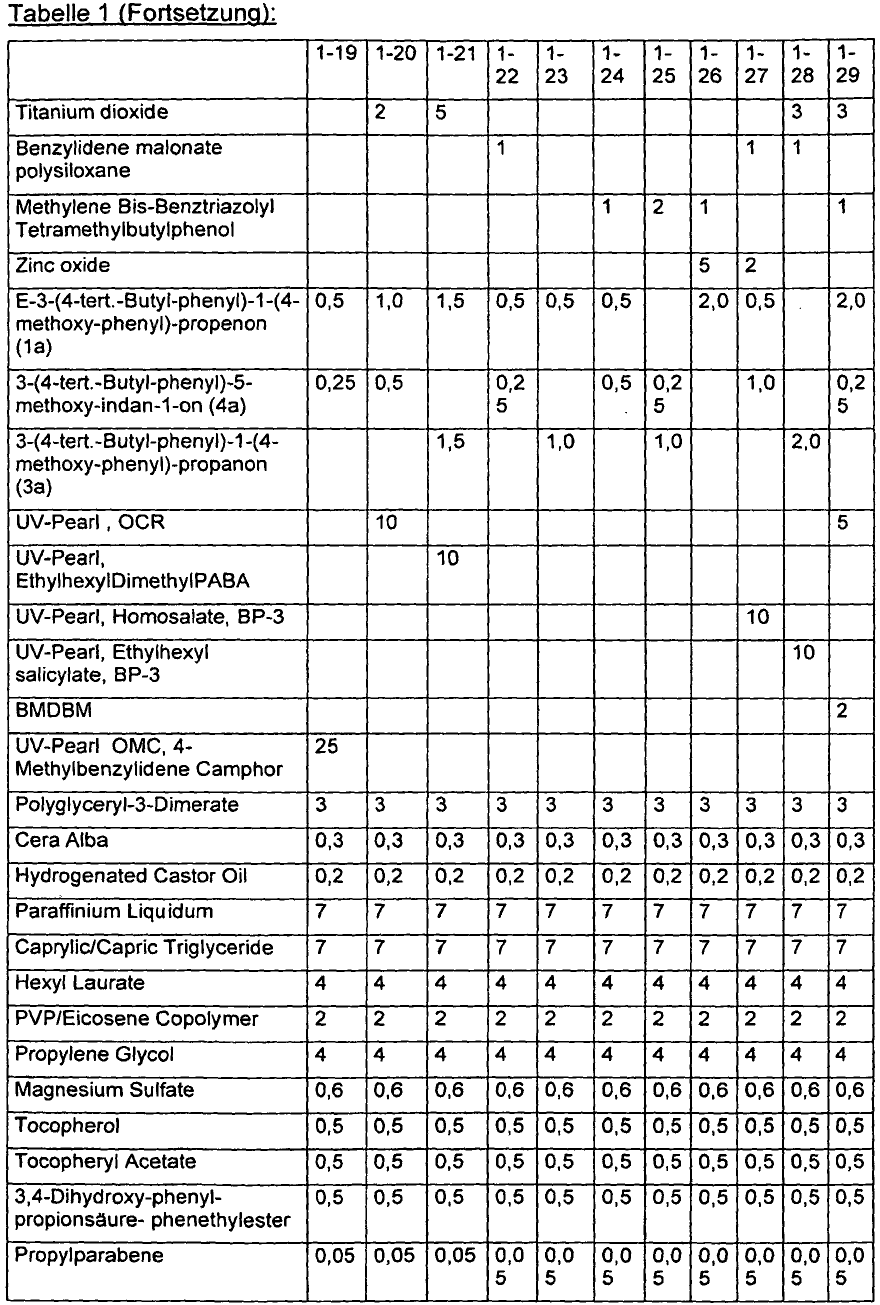

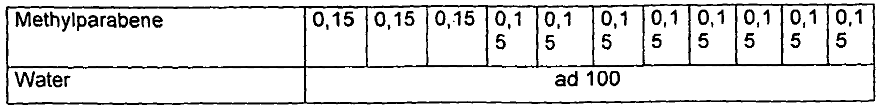

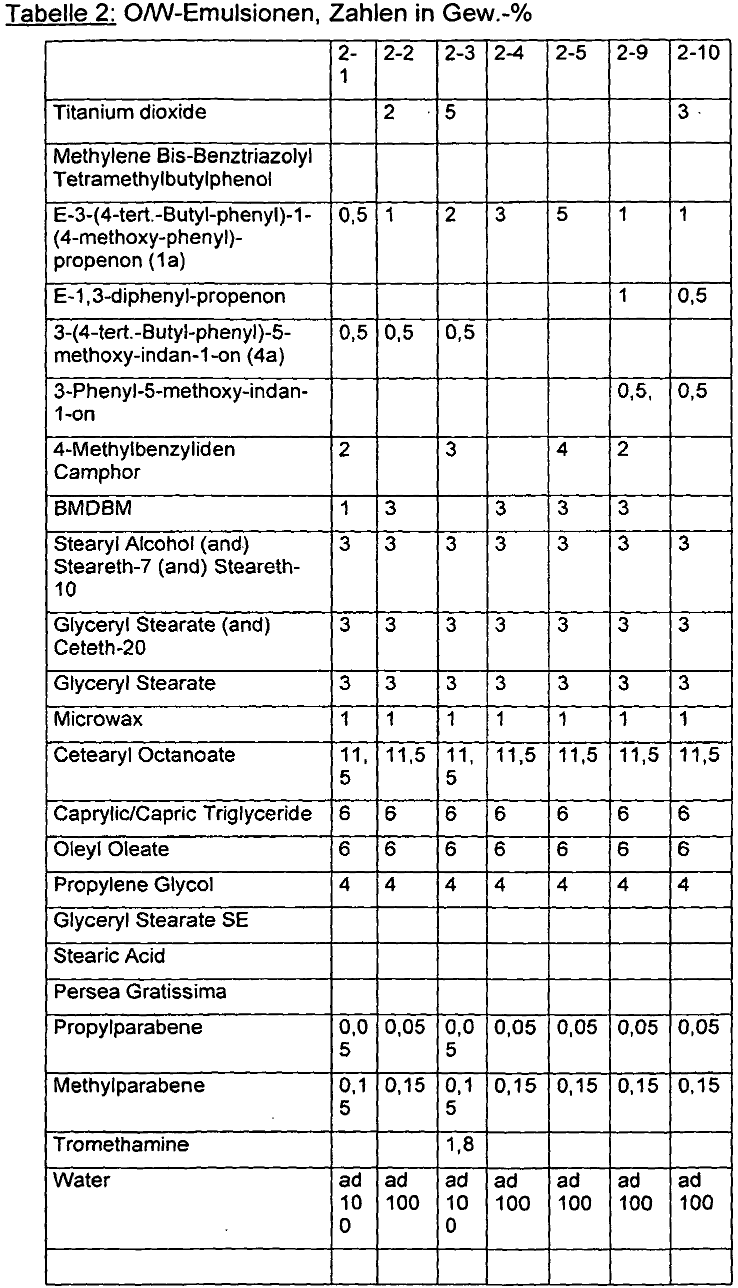

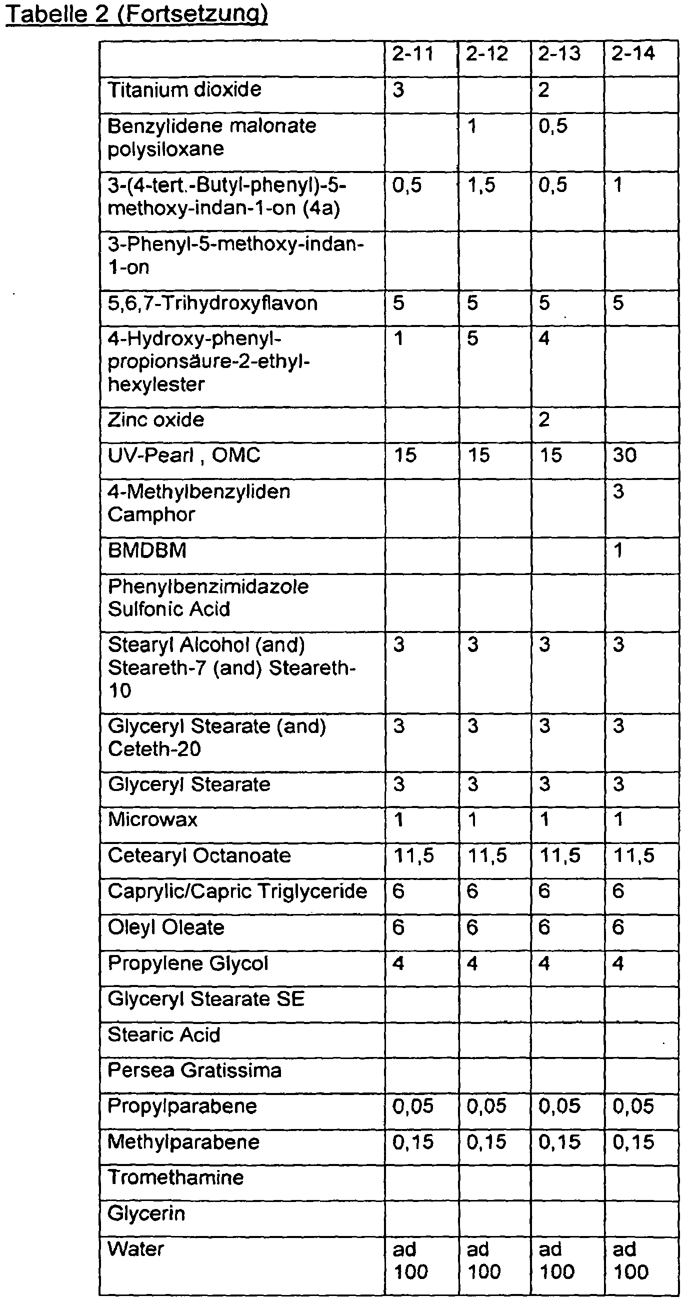

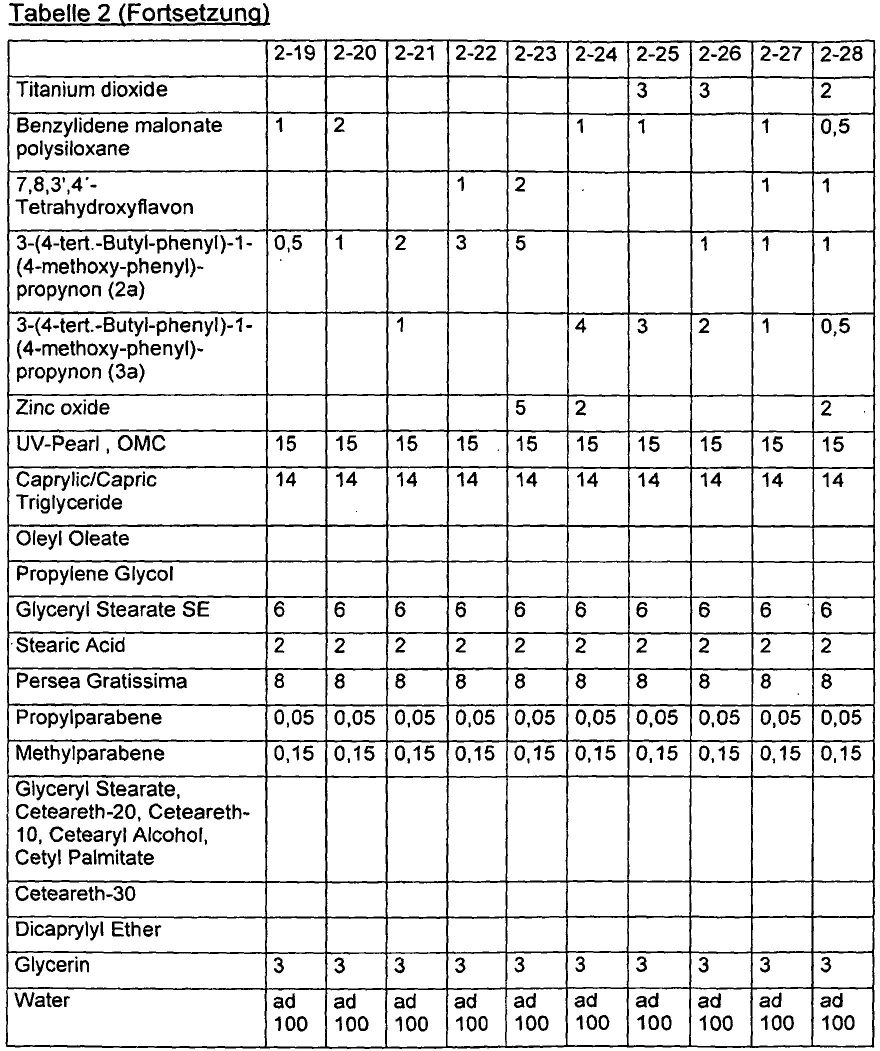

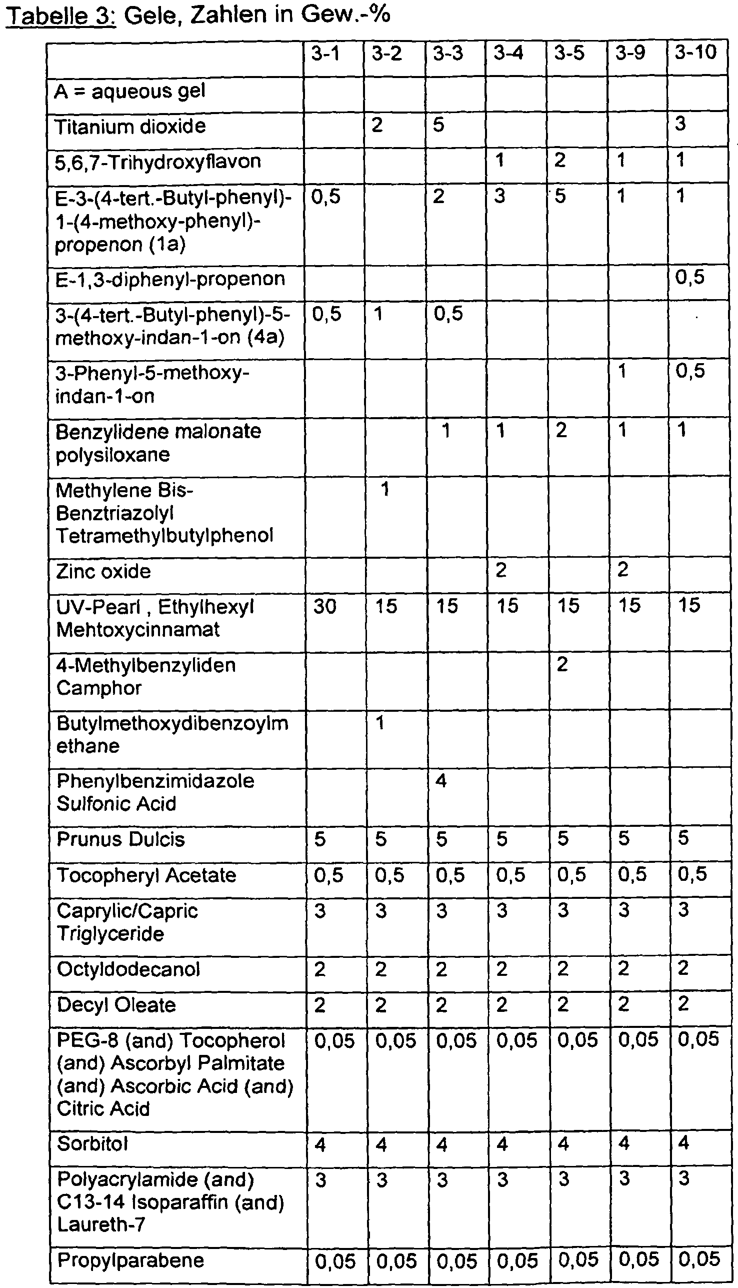

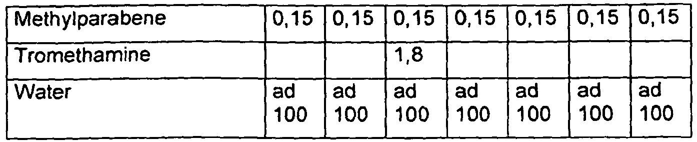

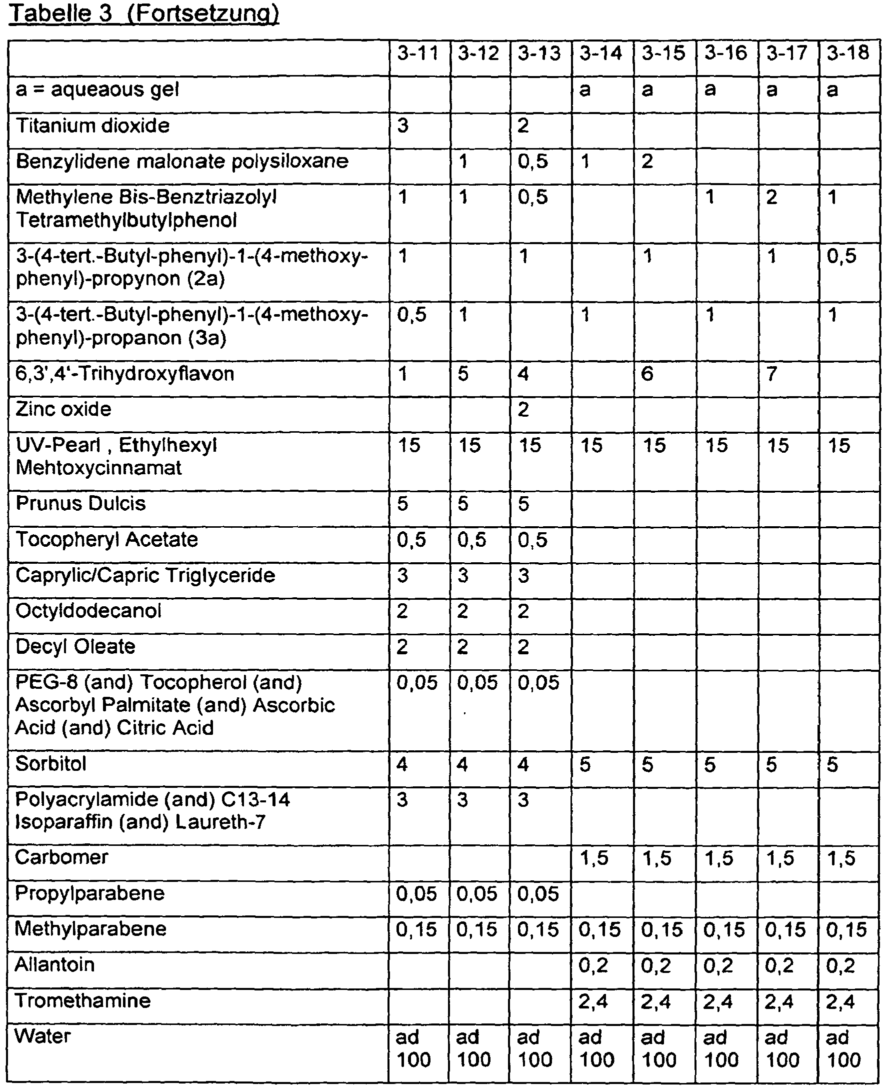

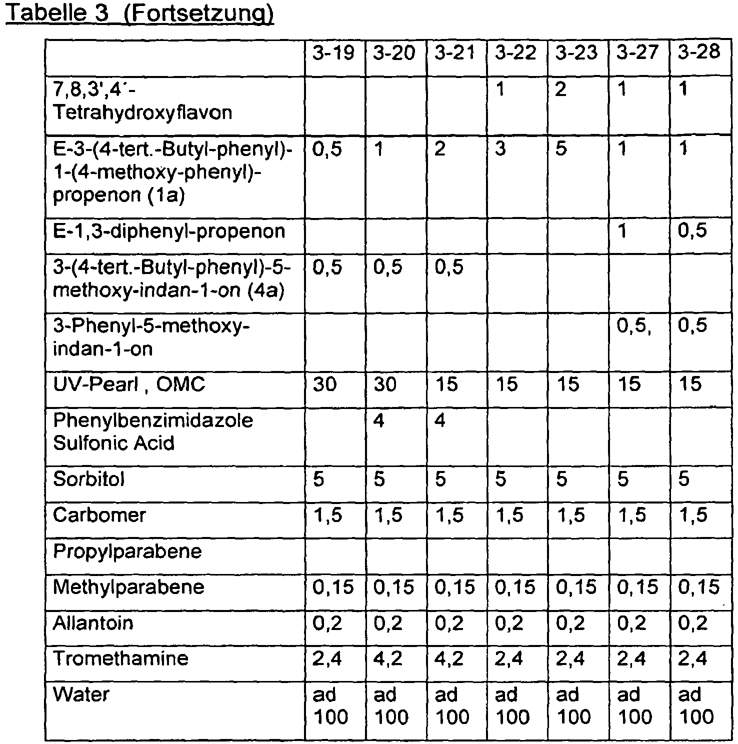

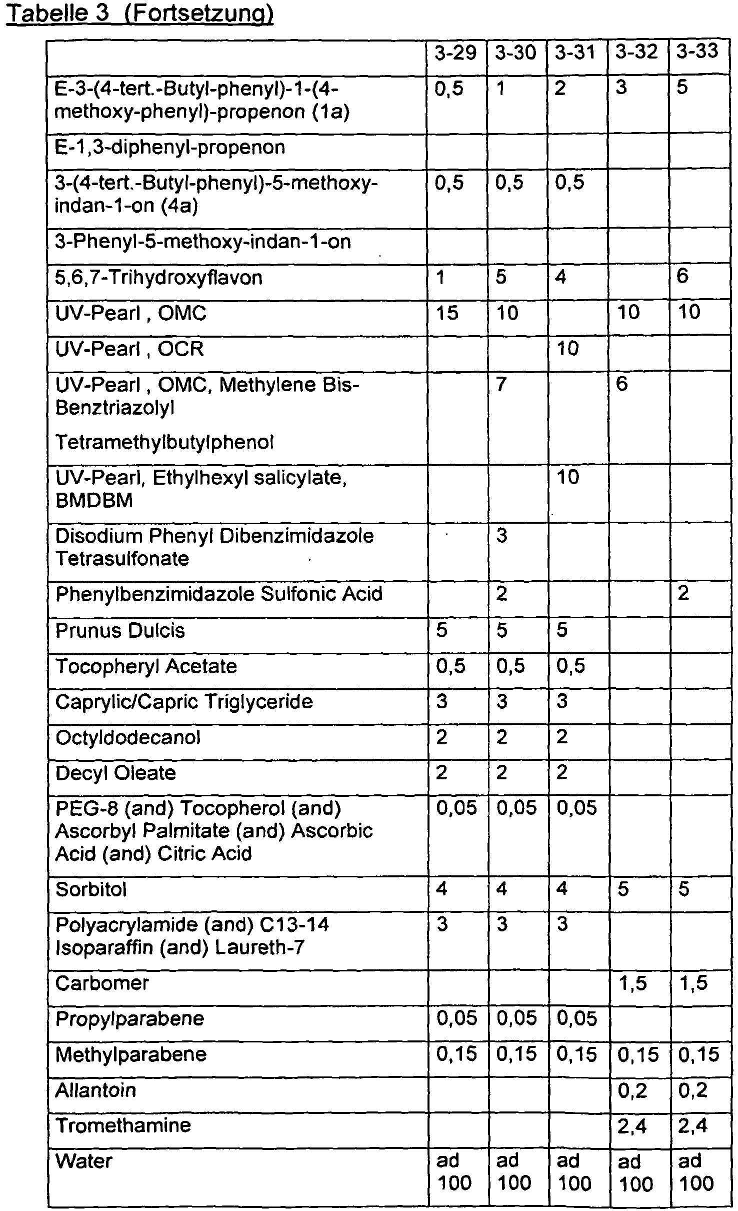

### Beispiel 5:

### Haarmascara

| Inhaltsstoffe | [%] |
|---|---|
| A | |
| PERLGLANZPIGMENT | 20,00 |
| B | |
| CETEARETH-25 | 1,80 |
| CETEARYLALCOHOL | 5,00 |
| DIMETHICONE | 1,00 |
| PHENOXYETHANOL, BUTYLPARABEN, ETHYLPARABEN, PROPYLPARABEN, METHYLPARABEN | 0,50 |
| C | |
| AQUA (WASSER) | ad 100 |
| POLYQUATERNIUM-16 | 3,0 |
| PROPYLENEGLYCOL | 1,80 |
| 3-(4-TERT.-BUTYL-PHENYL)-5-METHOXY-INDAN-1-ON (4a) | 0,25 |
| E-3-(4-TERT.-BUTYL-PHENYL)-1-(4-METHOXYPHENYL)-PROPENON (1a) | 1,5 |
| D | |
| AQUA (WASSER) | 9,50 |
| HYDROXYPROPYLCELLULOSE | 0,50 |
| E | |
| AQUA (WASSER) | 9,50 |
| MAGNESIUM ALUMINIUM SILICATE | 0,50 |
| IMIDAZOLIDINYL UREA | 0,30 |

### Herstellverfahren:

Phase B auf 75 °C, Phase C auf 80 °C erhitzen. Phase B langsam unter Rühren zu Phase C zugeben. Unter Rühren auf 65 °C abkühlen und homogenisieren. Auf 40 °C abkühlen und Phasen D, E und F zu Phase B/C unter Rühren zugeben und wiederum homogenisieren. Nun das Periglanzpigment unter Rühren zugeben. Auf Zimmertemperatur abkühlen und den pH-Wert auf 6,0-6,5 einstellen.

### Beispiel 6:

### Conditioner mit IR3535®

| Inhaltsstoffe | [%] |
|---|---|
| A | |
| ETHYLBUTYLACETYLAMINOPROPIONATE | 10,00 |
| PVP/VA COPOLYMER | 4,00 |
| PARFUM | 0,30 |
| QUATERNIUM-80 | 1,0 |
| PEG-40 HYDROGENATED CASTOR OIL | 1,00 |
| ALCOHOL | 15,00 |
| 3-(4-TERT.-BUTYL-PHENYL)-5-METHOXY-INDAN-1-ON (4a) | 0,5 |
| E-3-(4-TERT.-BUTYL-PHENYL)-1-(4-METHOXY-PHENYL)-PROPENON (1a) | 0,5 |
| B | |
| CETRIMONIUM CHLORIDE | 0,50 |
| AQUA (WASSER) | Ad 100 |
| C | |
| COCAMIDOPROPYL BETAINE | 4,00 |

### Herstellverfahren:

Phasen A und B separat vermischen. Phase B zu Phase A unter Rühren hinzugeben. Phase C zugeben.

Analog können Conditioner-Zubereitungen hergestellt werden, welche die folgenden Abwandlungen aufweisen:

| | |
|---|---|
| QUATERNIUM-80 | 2,0 |
| 3-(4-tert.-Butyl-phenyl)-1-(4-methoxy-phenyl)-propynon (2a) | 0,5 |

| | |
|---|---|
| QUATERNIUM-80 | 0 |
| 3-(4-tert.-Butyl-phenyl)-1-(4-methoxy-phenyl)-propanon (3a) | 0,5 |

## Patentansprüche

1. Nicht-therapeutische Verwendung einer oder mehrerer Verbindungen als Wirkstoffvorstufe in einer topischen Formulierung, **dadurch gekennzeichnet, dass** sich die Verbindung nach Applikation in einen Wirkstoff umwandelt, der in der Formulierung schlechter löslich ist als die Wirkstoffvorstufe,
**dadurch gekennzeichnet, dass** als Wirkstoffvorstufe eine Verbindung ausgewählt aus den Verbindungen der Formel 1, 2 und 3 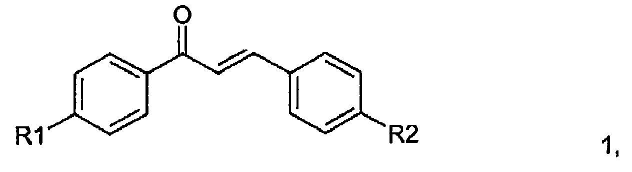 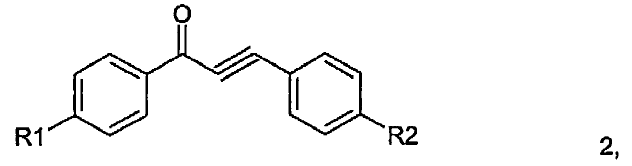 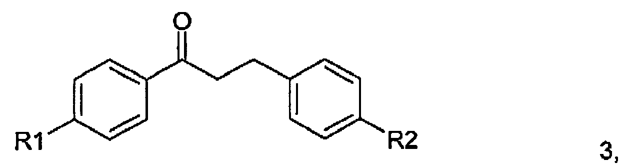 wobei R1 und R2 unabhängig voneinander für Methoxy, Isopropyl oder tert-Butyl stehen,
verwendet wird.

2. Verwendung nach Anspruch 1, **dadurch gekennzeichnet, dass** der Wirkstoff eine Verbindung der Formel 4 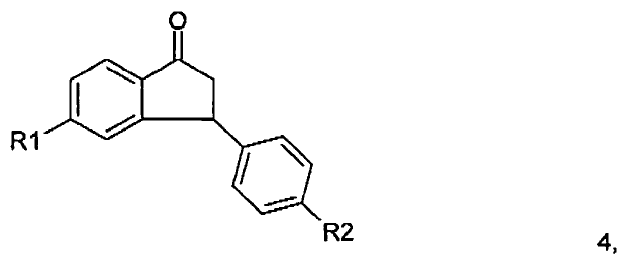 wobei R1 und R2 wie für Formel 1, 2 und 3 definiert sind,
ist.

3. Verwendung nach Anspruch 1 oder 2, **dadurch gekennzeichnet, dass** eine Verbindung ausgewählt aus den Verbindungen der Formel 1a-1d, 1k, 1m-1p, 2a-2d, 2k, 2m-2p, 3a-3d, 3k und 3m-3p
| | | | |
|---|---|---|---|
| 1a | 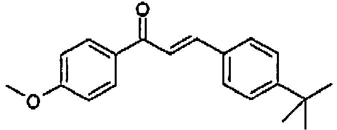 | 1b | 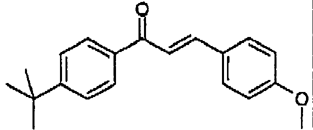 |
| 1c | 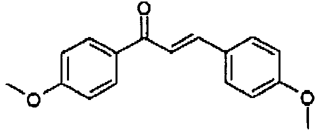 | 1d | 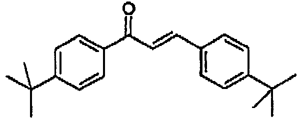 |
| 1k | 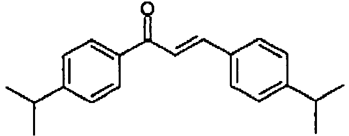 | | |
| 1m | 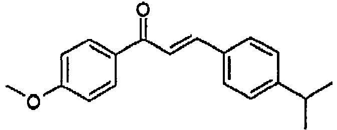 | 1n | 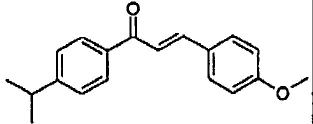 |
| 1o | 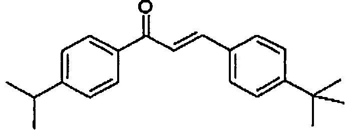 | 1p | 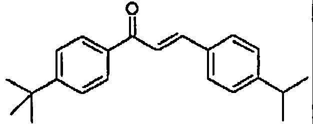 |
| | | | |
|---|---|---|---|
| 2a | 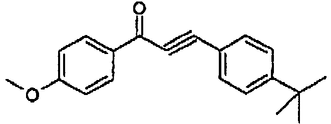 | 2b | 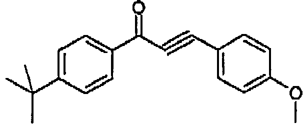 |
| 2c | 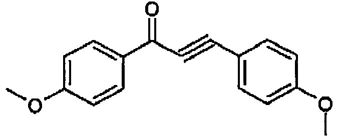 | 2d | 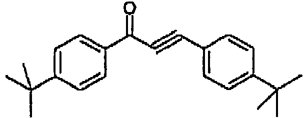 |
| 2k | 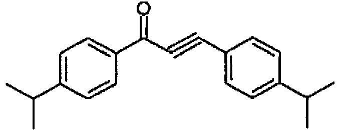 | | |
| 2m | 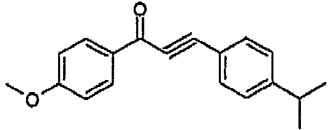 | 2n | 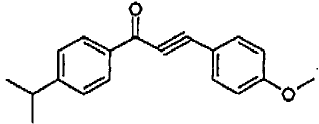 |
| 2o | 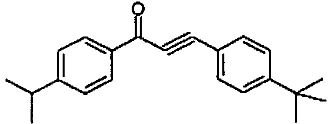 | 2p | 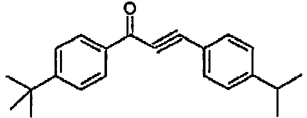 |
| | | | |
|---|---|---|---|
| 3a | 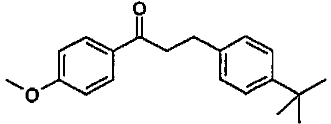 | 3b | 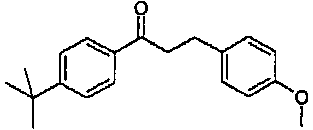 |
| 3c | 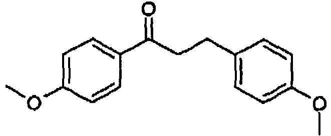 | 3d | 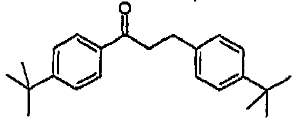 |
| 3k | 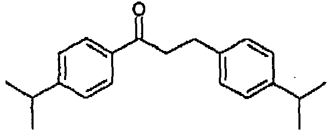 | | |
| 3m | 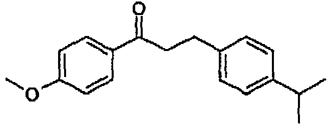 | 3n | 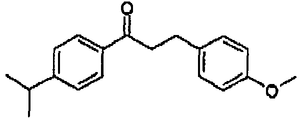 |
| 3o | 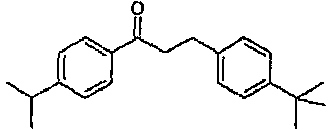 | 3p | 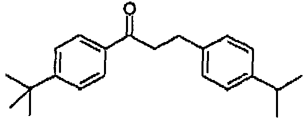 |
verwendet wird.

4. Verwendung nach Anspruch 3, **dadurch gekennzeichnet, dass** die Verbindung der Formel 1a verwendet wird.

5. Verwendung nach einem oder mehreren der Ansprüche 2 bis 3, **dadurch gekennzeichnet, dass** der Wirkstoff der Formel 4 ausgewählt ist aus den Verbindungen der Formel 4a-4d, 4k, 4m-4p
| | | | |
|---|---|---|---|
| 4a | 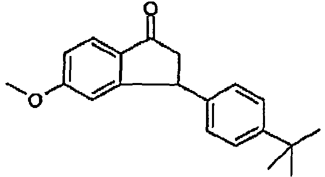 | 4b | 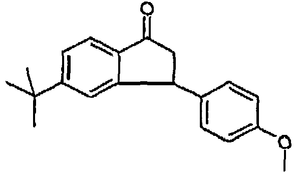 |
| 4c | 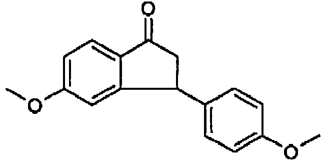 | 4d | 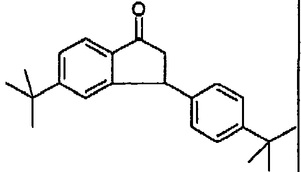 |
| 4k | 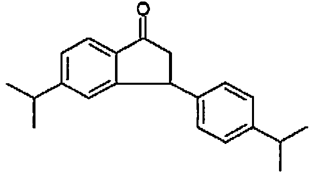 | | |
| 4m | 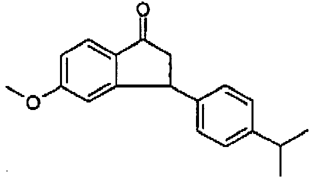 | 4n | 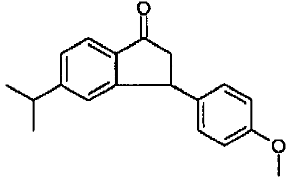 |
| 4o | 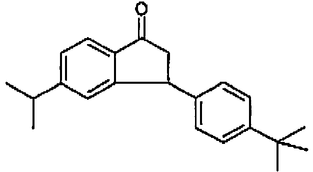 | 4p | 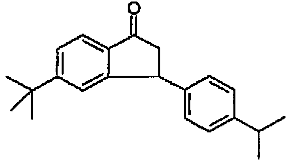 |

6. Kosmetische Zubereitung enthaltend mindestens eine Verbindung der Formel 1, 2 und/oder 3 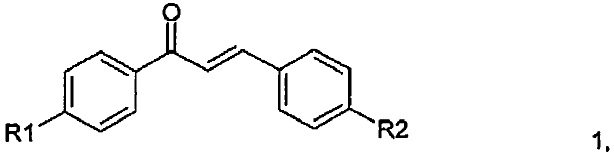 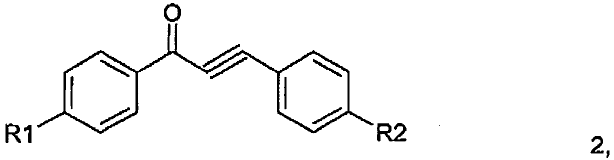 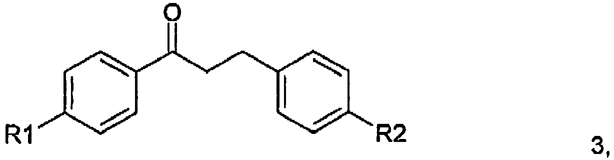 wobei R1 und R2 unabhängig voneinander für Methoxy, Isopropyl oder tert-Butyl stehen
und wobei sich die mindestens eine Verbindung der Formel 1, 2 und/oder 3 nach Applikation in einen Wirkstoff umwandelt, der in der Formulierung schlechter löslich ist als die mindestens eine Verbindung der Formel 1, 2 und/oder 3.

7. Zubereitung nach Anspruch 6, **dadurch gekennzeichnet, dass** die mindestens eine Verbindung der Formel 1, 2 und/oder 3 in Mengen von 0,05 bis 10 Gew% enthalten ist.

8. Zubereitung nach Anspruch 6 oder 7, **dadurch gekennzeichnet, dass** sie Selbstbräunungssubstanzen und/ oder UV-Filter enthält.

9. Zubereitung nach einem oder mehreren der Ansprüche 6 bis 8, **dadurch gekennzeichnet, dass** sie ein oder mehrere Antioxidantien und/oder ein oder mehrere Vitamine enthält.

10. Zubereitung nach einem oder mehreren der Ansprüche 6 bis 9, **dadurch gekennzeichnet, dass** sie ein oder mehrere Verbindungen der Formel 5a und/oder 5b 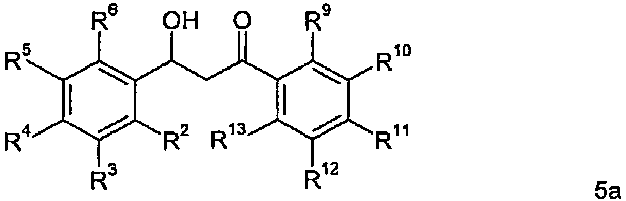 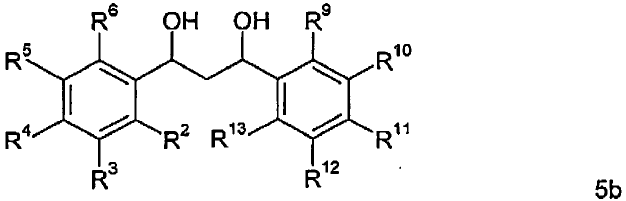 wobei
R⁴ und R¹¹ jeweils unabhängig voneinander H, geradkettige oder verzweigte C1- bis C20-Alkylgruppe, geradkettige oder verzweigte C1-bis C20-Alkoxygruppe oder geradkettige oder verzweigte C1- bis C20-Dialkylaminogruppe,
R⁶ H oder eine Carbonsäure-, Phosphorsäure-, Sutfonsäure-, Schwefelsäure- oder Sulfonfunktion, die mit geradkettigen oder verzweigten C1- bis C20-Alkylgruppen oder geradkettigen oder verzweigten C3- bis C20-Alkenylgruppen verestert oder alkyliert sein kann und
R², R³, R⁵, R⁹, R¹⁰, R¹² und R¹³ H bedeuten,
oder deren Salze enthält.

11. Zubereitung nach Anspruch 10, **dadurch gekennzeichnet, dass** die ein oder mehrere Verbindungen der Formel 5a und 5b ausgewählt sind aus den Verbindungen umfassend 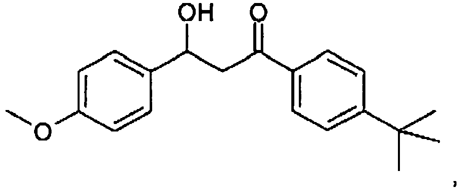 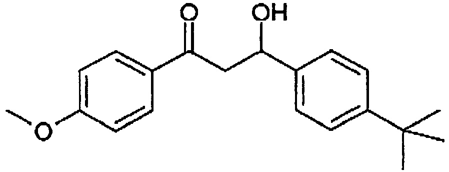 und

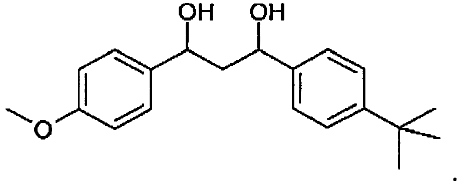

12. Verfahren zur Herstellung einer Zubereitung nach einem oder mehreren der Ansprüche 6 bis 11, **dadurch gekennzeichnet, dass** mindestens eine Verbindung der Formel 1, 2 und/oder 3 mit einem Träger und gegebenenfalls mit weiteren Aktiv- oder Hilfsstoffen vermischt wird.

13. Verbindung, ausgewählt aus den Verbindungen umfassend
| | | | |
|---|---|---|---|
| 2a | 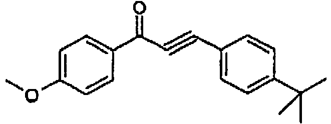 | 2b | 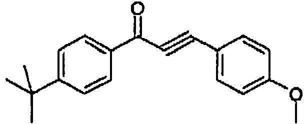 |
| 2k | 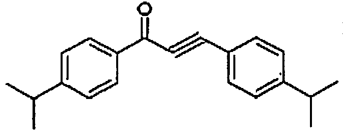 | 2m | 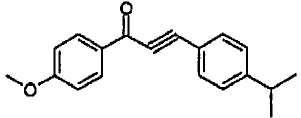 |
| | | | |
|---|---|---|---|
| 2n | 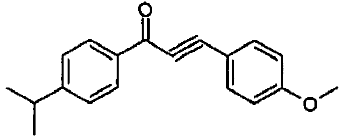 | 2o | 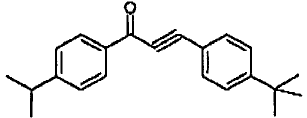 |
| 2p | 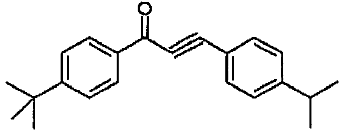 | | |
| | | | |
|---|---|---|---|
| 3d | 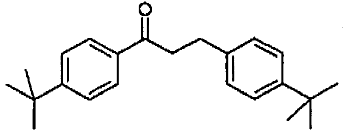 | 3k | 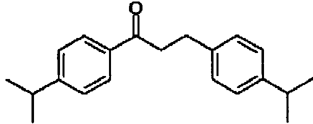 |
| 3o | 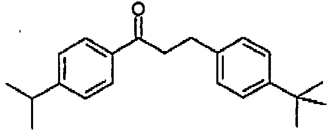 | 3p | 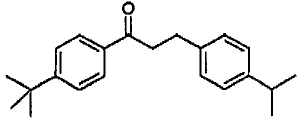 |
| | | | |
|---|---|---|---|
| 4a | 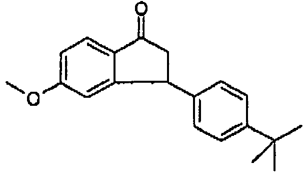 | 4b | 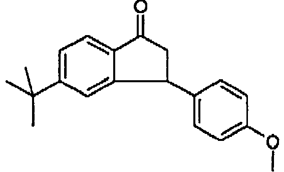 |
| 4d | 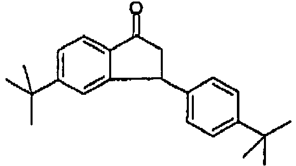 | | |
| | | 4k | 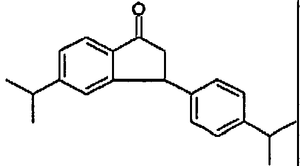 |
| 4m | 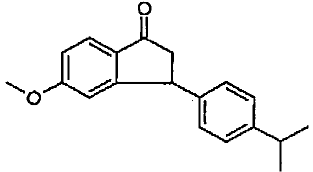 | 4n | 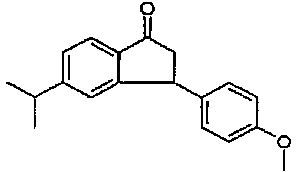 |
| 4o | 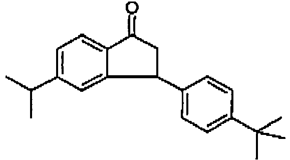 | 4p | 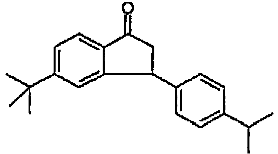 |

## Claims

1. Non-therapeutic use of one or more compounds as active compound precursor in a topical formulation, **characterised in that**, after application, the compound is converted into an active compound which has worse solubility than the active compound precursor in the formulation, **characterised in that** the active compound precursor used is a compound selected from the compounds of the formulae 1, 2 and 3, 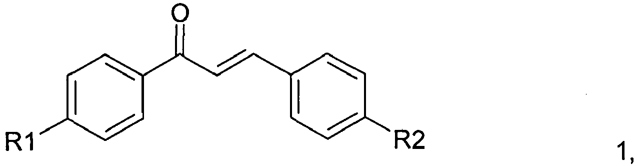 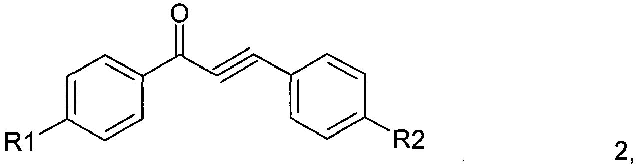 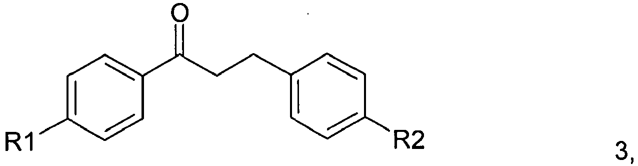 where R1 and R2 stand, independently of one another, for methoxy, isopropyl or tert-butyl.

2. Use according to Claim 1, **characterised in that** the active compound is a compound of the formula 4, 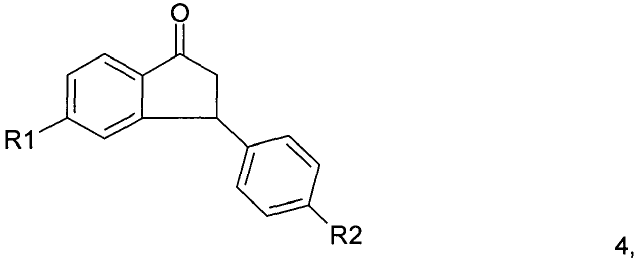 where R1 and R2 are as defined for formulae 1, 2 and 3.

3. Use according to Claim 1 or 2, **characterised in that** a compound selected from the compounds of the formulae 1a-1d, 1k, 1m-1p, 2a-2d, 2k, 2m-2p, 3a-3d, 3k and 3m-3p
| | | | |
|---|---|---|---|
| 1a | 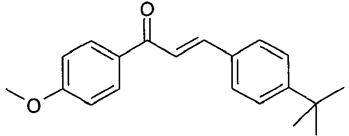 | 1b | 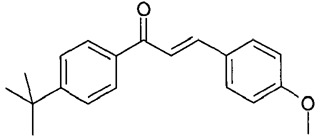 |
| 1c | 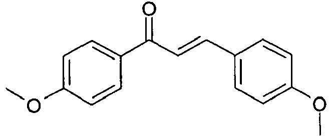 | 1d | 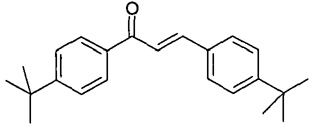 |
| 1k | 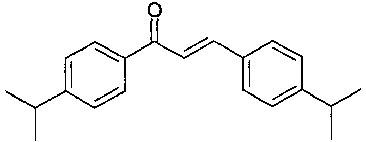 | | |
| 1m | 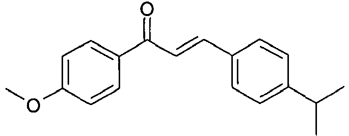 | 1n | 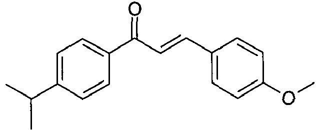 |
| 1o | 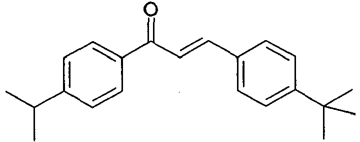 | 1p | 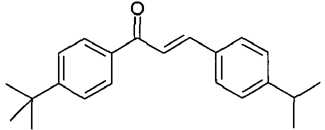 |
| 2a | 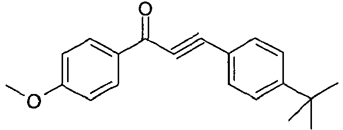 | 2b | 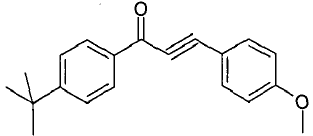 |
| 2c | 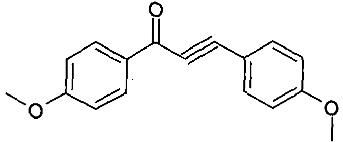 | 2d | 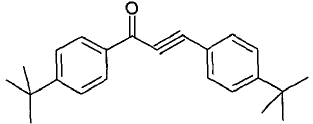 |
| 2k | 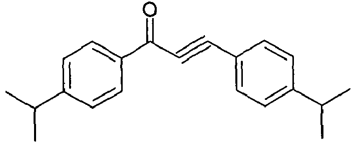 | | |
| 2m | 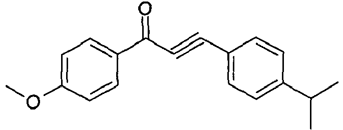 | 2n | 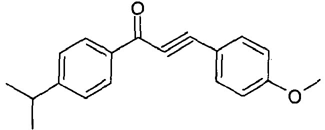 |
| 2o | 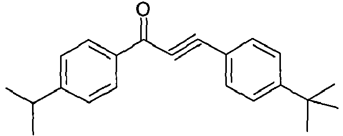 | 2p | 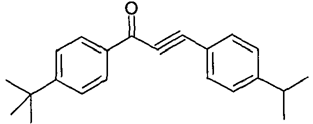 |
| | | | |
|---|---|---|---|
| 3a | 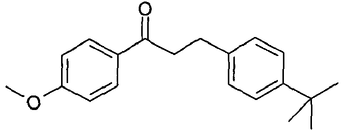 | 3b | 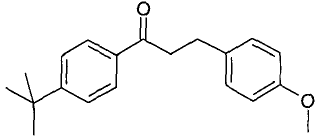 |
| 3c | 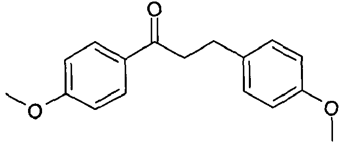 | 3d | 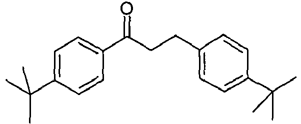 |
| 3k | 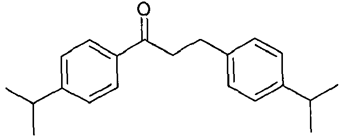 | | |
| 3m | 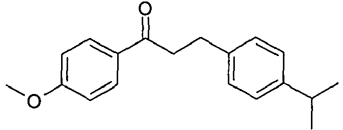 | 3n | 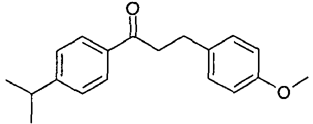 |
| 3o | 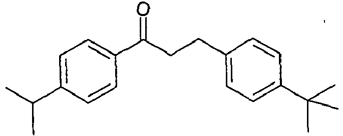 | 3p | 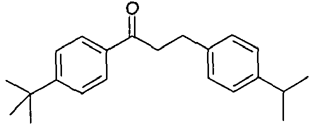 |
is used.

4. Use according to Claim 3, **characterised in that** the compound of the formula 1a is used.

5. Use according to one or more of Claims 2 to 3, **characterised in that** the active compound of the formula 4 is selected from the compounds of the formulae 4a-4d, 4k and 4m-4p,
| | | | |
|---|---|---|---|
| 4a | 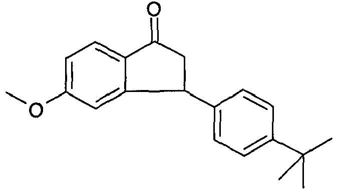 | 4b | 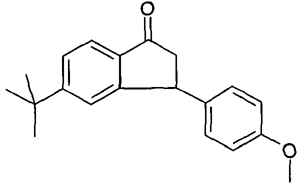 |
| 4c | 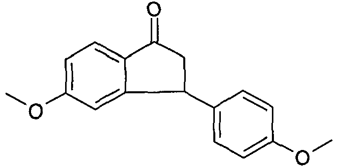 | 4d | 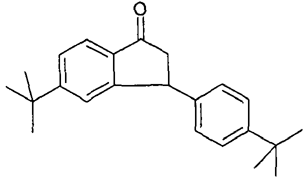 |
| 4k | 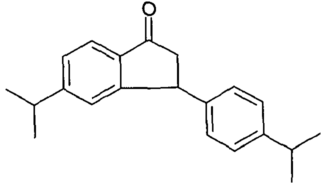 | | |
| 4m | 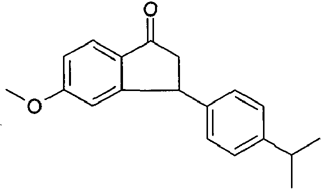 | 4n | 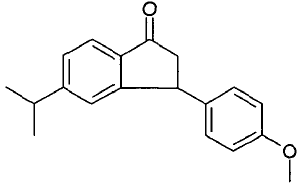 |
| 4o | 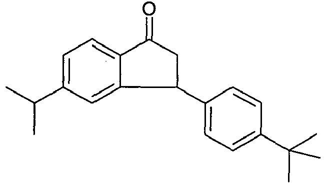 | 4p | 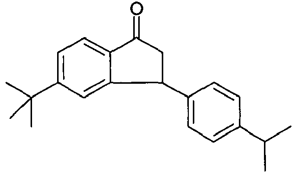 |

6. Cosmetic preparation comprising at least one compound of the formula 1, 2 and/or 3, 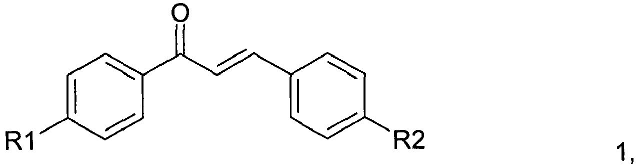 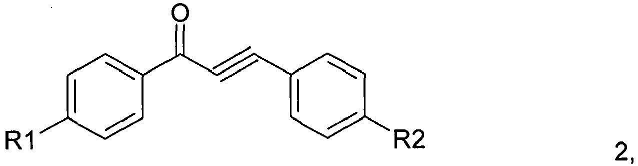 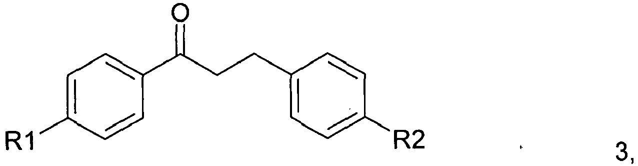 where R1 and R2 stand, independently of one another, for methoxy, isopropyl or tert-butyl,
and where, after application, the at least one compound of the formula 1, 2 and/or 3 is converted into an active compound which has worse solubility than the at least one compound of the formula 1, 2 and/or 3 in the formulation.

7. Preparation according to Claim 6, **characterised in that** the at least one compound of the formula 1, 2 and/or 3 is present in amounts of 0.05 to 10% by weight.

8. Preparation according to Claim 6 or 7, **characterised in that** it comprises self-tanning substances and/or UV filters.

9. Preparation according to one or more of Claims 6 to 8, **characterised in that** it comprises one or more antioxidants and/or one or more vitamins.

10. Preparation according to one or more of Claims 6 to 9, **characterised in that** it comprises one or more compounds of the formula 5a and/or 5b, 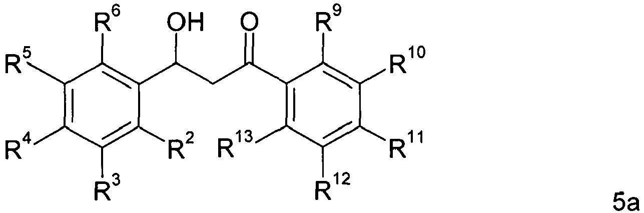 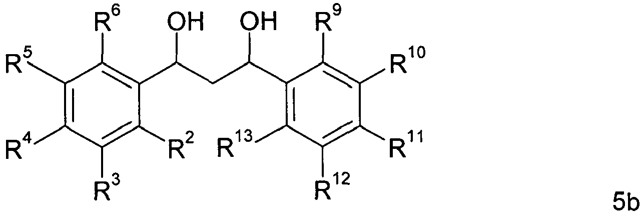 where
R⁴ and R¹¹ each, independently of one another, denote H, a straight-chain or branched C1- to C20-alkyl group, a straight-chain or branched C1- to C20-alkoxy group or a straight-chain or branched C1- to C20-dialkylamino group,
R⁶ denotes H or a carboxylic acid, phosphoric acid, sulfonic acid, sulfuric acid or sulfone function, which may be esterified or alkylated with straight-chain or branched C1- to C20-alkyl groups or straight-chain or branched C3- to C20-alkenyl groups, and
R², R³, R⁵, R⁹, R¹⁰, R¹² and R¹³ denote H,
or salts thereof.

11. Preparation according to Claim 10, **characterised in that** the one or more compounds of the formulae 5a and 5b are selected from the compounds comprising 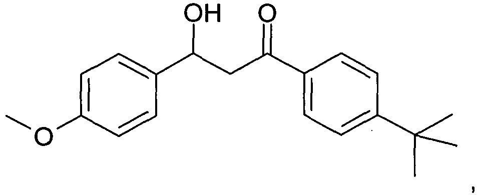 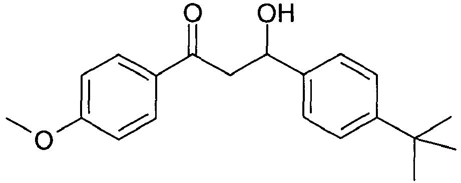 and

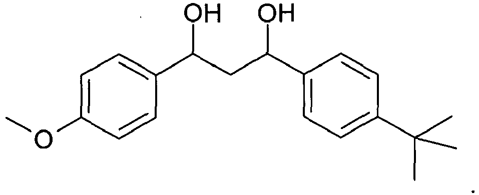

12. Process for the preparation of a preparation according to one or more of Claims 6 to 11, **characterised in that** at least one compound of the formula 1, 2 and/or 3 is mixed with an excipient and optionally with further active compounds or adjuvants.

13. Compound selected from the compounds comprising
| | | | |
|---|---|---|---|
| 2a | 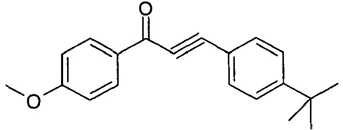 | 2b | 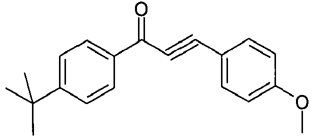 |
| 2k | 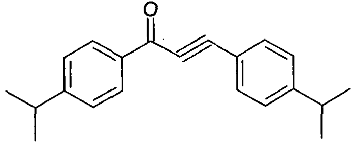 | 2m | 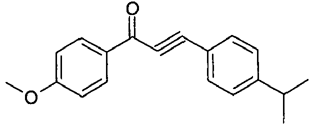 |
| 2n | 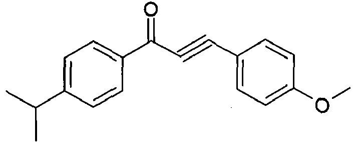 | 2o | 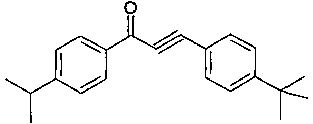 |
| 2p | 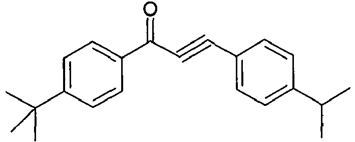 | | |
| | | | |
|---|---|---|---|
| 3d | 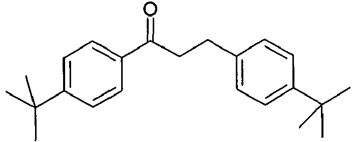 | 3k | 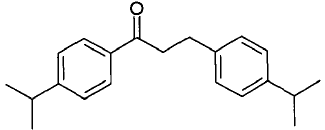 |
| 3o | 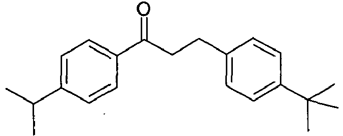 | 3p | 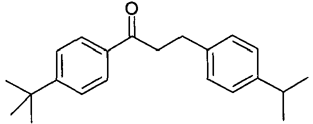 |
| | | | |
|---|---|---|---|
| 4a | 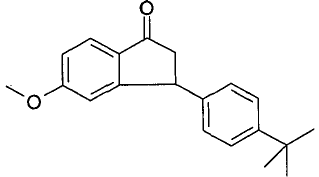 | 4b | 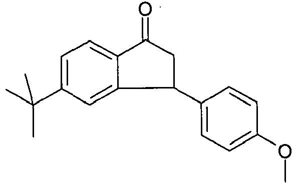 |
| 4d | 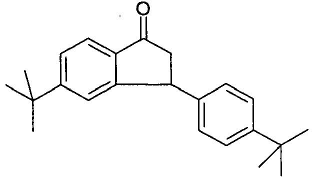 | | |
| | | 4k | 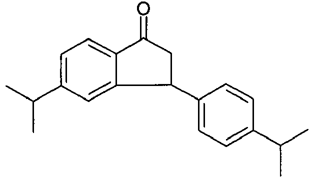 |
| 4m | 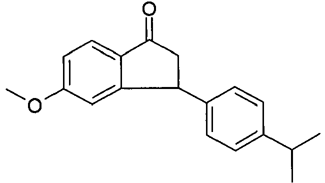 | 4n | 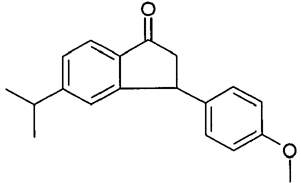 |
| 4o | 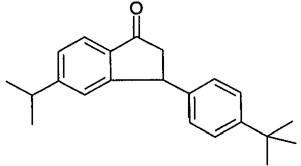 | 4p | 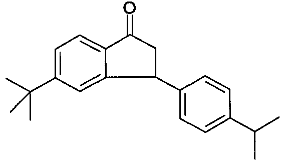 |

## Revendications

1. Utilisation non thérapeutique d'un ou plusieurs composés comme précurseur de composé actif dans une formulation topique, **caractérisée en ce que**, après application, le composé est converti en composé actif ayant une solubilité moindre que le précurseur de composé actif dans la formulation,
**caractérisée en ce que** le précurseur de composé actif utilisé est un composé choisi parmi les composés de formules 1, 2 et 3, 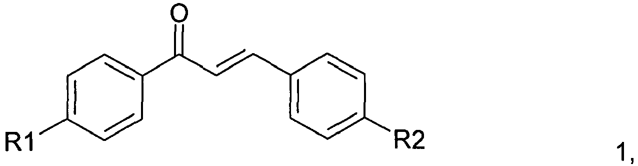 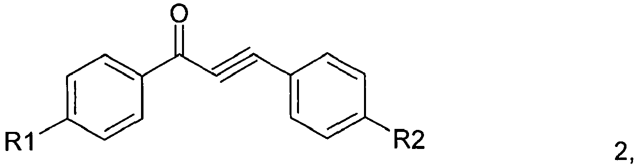 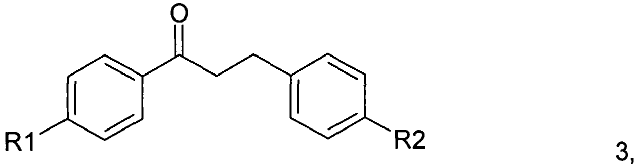 où R1 et R2 représentent, indépendamment l'un de l'autre, méthoxy, isopropyle ou tertio-butyle.

2. Utilisation selon la revendication 1, **caractérisée en ce que** le composé actif est un composé de formule 4, 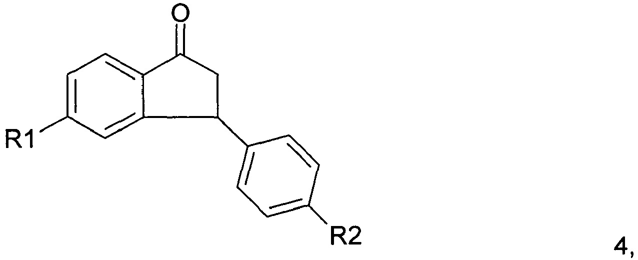 où R1 et R2 sont tels que définis pour les formules 1, 2 et 3.

3. Utilisation selon la revendication 1 ou 2, **caractérisée en ce qu'**un composé choisi parmi les composés de formules 1a-1d, 1k, 1m-1p, 2a-2d, 2k, 2m-2p, 3a-3d, 3k et 3m-3p
| | | | |
|---|---|---|---|
| 1a | 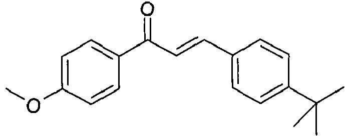 | 1b | 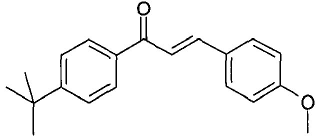 |
| 1c | 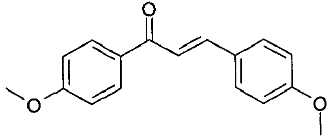 | 1d | 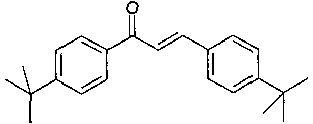 |
| 1k | 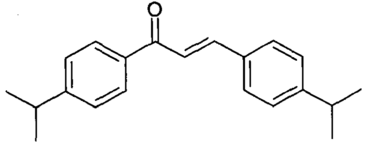 | | |
| 1m | 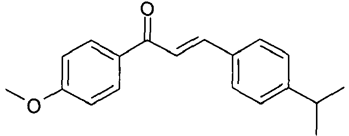 | 1n | 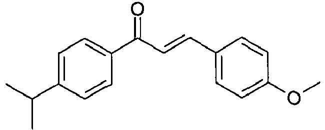 |
| 1o | 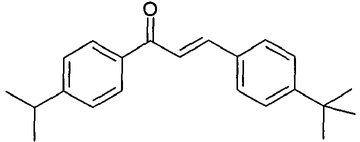 | 1p | 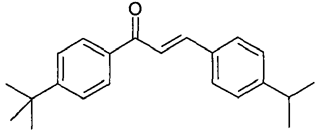 |
| 2a | 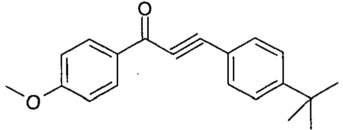 | 2b | 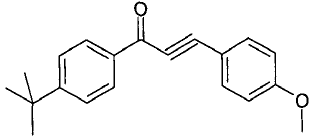 |
| 2c | 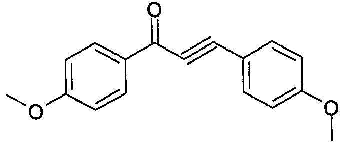 | 2d | 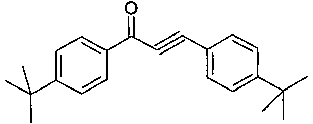 |
| 2k | 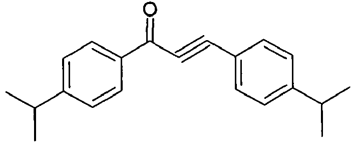 | | |
| 2m | 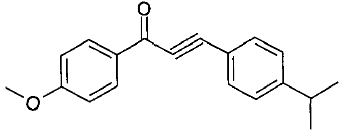 | 2n | 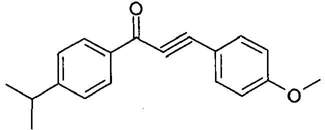 |
| 2o | 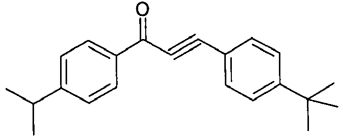 | 2p | 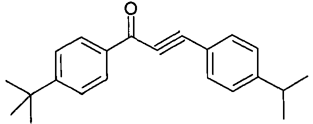 |
| | | | |
|---|---|---|---|
| 3a | 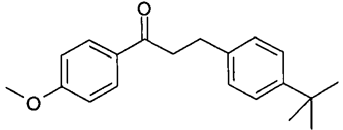 | 3b | 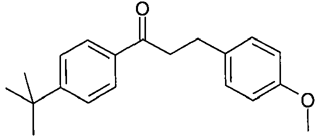 |
| 3c | 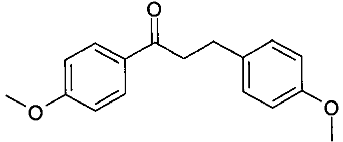 | 3d | 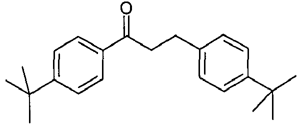 |
| 3k | 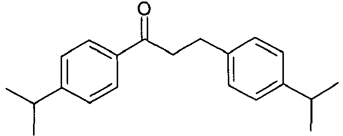 | | |
| 3m | 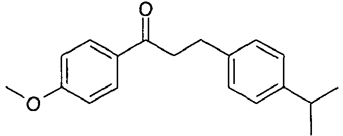 | 3n | 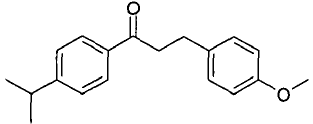 |
| 3o | 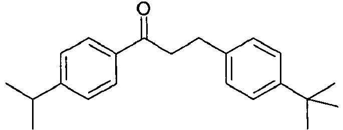 | 3p | 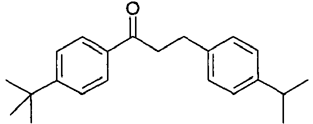 |
est utilisé.

4. Utilisation selon la revendication 3, **caractérisée en ce que** le composé de formule 1 a est utilisé.

5. Utilisation selon l'une ou plusieurs parmi les revendications 2 à 3, **caractérisée en ce que** le composé actif de formule 4 est choisi parmi les composés de formules 4a-4d, 4k et 4m-4p,
| | | | |
|---|---|---|---|
| 4a | 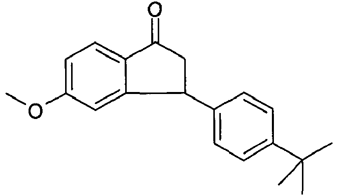 | 4b | 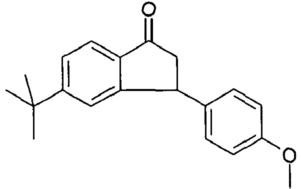 |
| 4c | 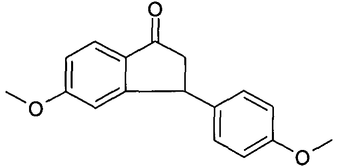 | 4d | 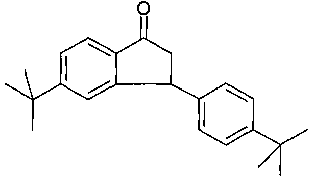 |
| 4k | 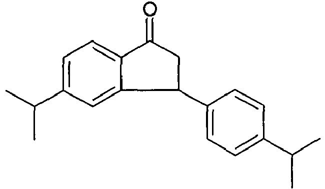 | | |
| 4m | 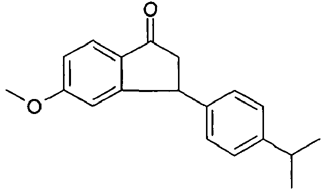 | 4n | 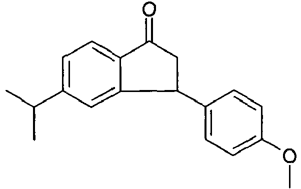 |
| 4o | 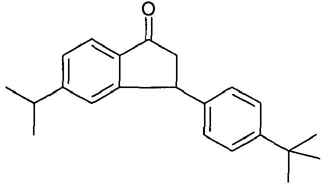 | 4p | 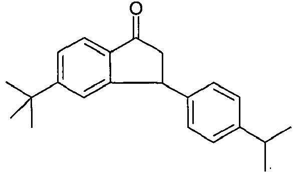 |

6. Préparation cosmétique comprenant au moins un composé de formule 1, 2 et/ou 3, 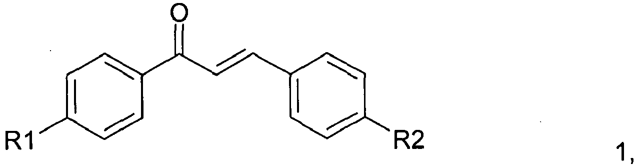 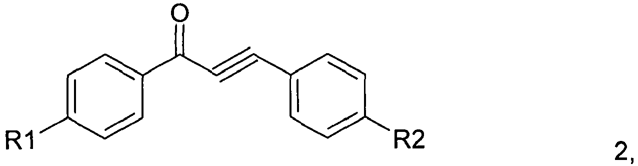 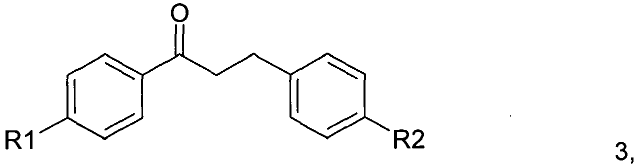 où R1 et R2 représentent, indépendamment l'un de l'autre, méthoxy, isopropyle ou tertio-butyle,
et où, après application, le au moins un composé de formule 1, 2 et/ou 3 est converti en composé actif ayant une solubilité moindre que le au moins un composé de formule 1, 2 et/ou 3 dans la formulation.

7. Préparation selon la revendication 6, **caractérisée en ce que** le au moins un composé de formule 1, 2 et/ou 3 est présent selon des quantités allant de 0,05 à 10% en poids.

8. Préparation selon la revendication 6 ou 7, **caractérisée en ce qu'**elle comprend des substances auto-bronzantes et/ou des filtres anti-UV.

9. Préparation selon l'une ou plusieurs parmi les revendications 6 à 8, **caractérisée en ce qu'**elle comprend un ou plusieurs antioxydants et/ou une ou plusieurs vitamines.

10. Préparation selon l'une ou plusieurs parmi les revendications 6 à 9, **caractérisée en ce qu'**elle comprend un ou plusieurs composés de formule 5a et/ou 5b, 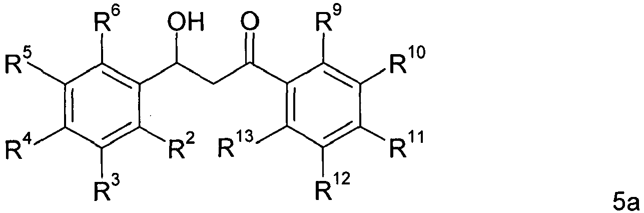 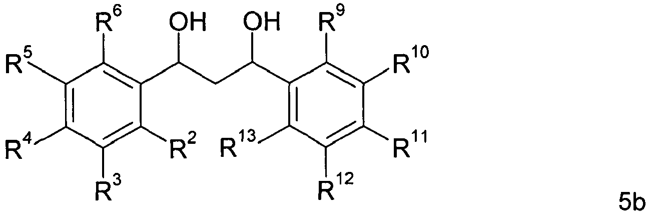 où
R⁴ et R¹¹ représentent chacun, indépendamment l'un de l'autre, H, un groupement C1- à C20-alkyle à chaîne linéaire ou ramifiée, un groupement C1- à C20-alcoxy à chaîne linéaire ou ramifiée ou un groupement C1- à C20-dialkylamino à chaîne linéaire ou ramifiée,
R⁶ désigne H ou une fonction acide carboxylique, acide phosphorique, acide sulfonique, acide sulfurique ou sulfone, pouvant être estérifiée ou alkylée par des groupements C1- à C20-alkyle à chaîne linéaire ou ramifiée ou des groupements C3- à C20-alcényle à chaîne linéaire ou ramifiée, et
R², R³, R⁵, R⁹, R¹⁰, R¹² et R¹³ désignent H,
ou des sels de ceux-ci.

11. Préparation selon la revendication 10, **caractérisée en ce que** les un ou plusieurs composés de formules 5a et 5b sont choisis parmi les composés comprenant 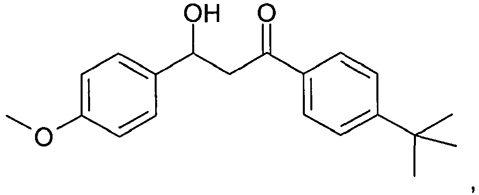 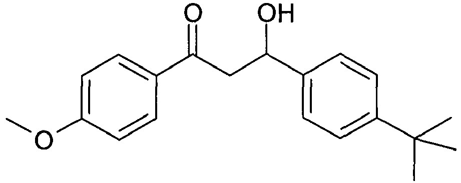 et

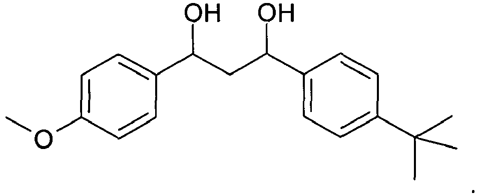

12. Procédé de préparation d'une préparation selon l'une ou plusieurs parmi les revendications 6 à 11, **caractérisé en ce qu'**au moins un composé de formule 1, 2 et/ou 3 est mélangé avec un excipient et éventuellement avec d'autres composés actifs ou adjuvants.

13. Composé choisi parmi les composés comprenant
| | | | |
|---|---|---|---|
| 2a | 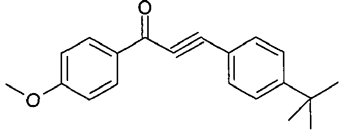 | 2b | 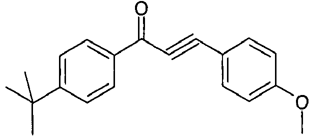 |
| 2k | 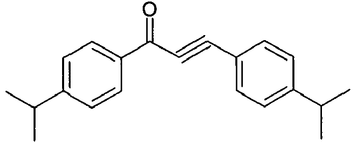 | 2m | 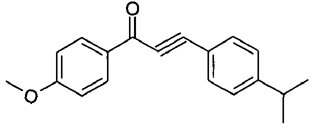 |
| 2n | 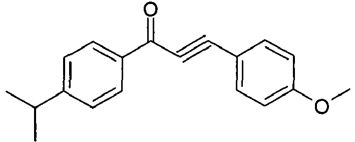 | 2o | 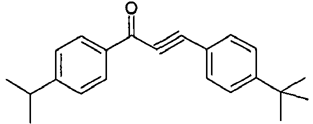 |
| 2p | 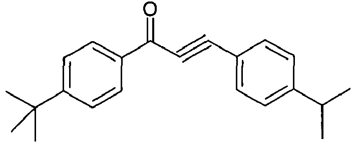 | | |
| | | | |
|---|---|---|---|
| 3d | 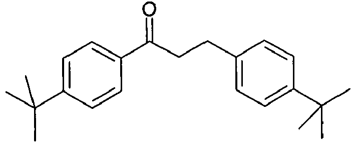 | 3k | 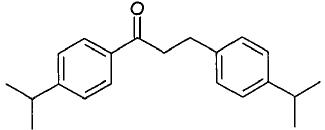 |
| 3o | 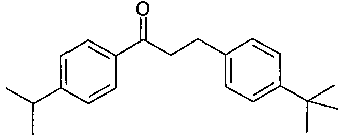 | 3p | 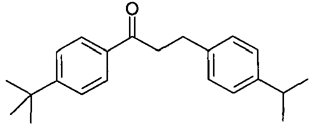 |
| | | | |
|---|---|---|---|
| 4a | 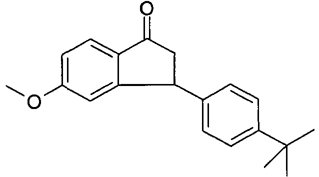 | 4b | 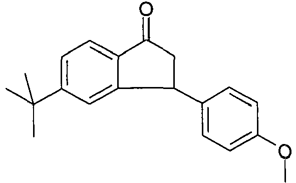 |
| | | | |
|---|---|---|---|
| 4d | 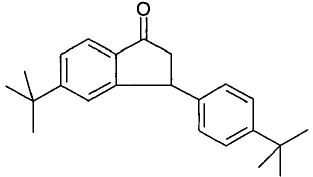 | | |
| | | 4k | 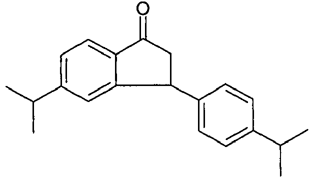 |
| 4m | 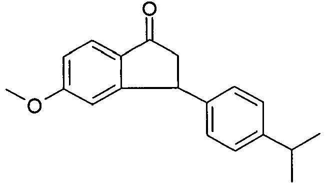 | 4n | 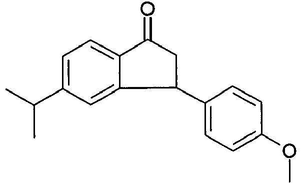 |
| 4o | 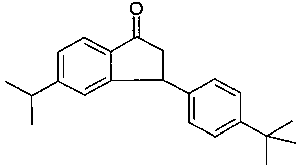 | 4p | 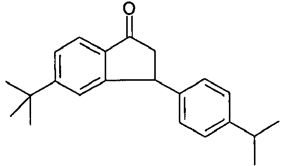 |
